# EUROPÄISCHE PATENTSCHRIFT

(11) **EP 2 245 986 B1**
(45) Veröffentlichungstag und Bekanntmachung des Hinweises auf die Patenterteilung: **16.10.2013**
(21) Anmeldenummer: 10170275.1
(22) Anmeldetag: 22.08.2008
(51) Int. Cl.: A61B 6/00

(54) **Pyramidenförmige Röntgenmarkervorrichtung**
Arrangement of x-ray markers in the form of a pyramid
Arrangement de marqueurs de radiographie en forme de pyramide

(43) Veröffentlichungstag der Anmeldung: 03.11.2010
(62) Teilanmeldung aus: 08162808.3
(73) Patentinhaber: BrainLAB AG, 85622 Feldkirchen (DE)
(72) Erfinder: Weiser, Manfred, 81825, München (DE); Heigl, Rupert, 81379, München (DE)
(74) Vertreter: Schwabe - Sandmair - Marx

(56) Entgegenhaltungen:
- EP-A- 0 853 920
- DE-A1- 19 703 556
- US-A- 6 044 132
- US-A1- 2005 109 855
- US-A1- 2005 113 682
- US-A1- 2007 122 020
- SCHÖNHERR ET AL: "Ein neues algorithmisches Verfahren zur Fluoroskopie-basierten Neuronavigation" INFORMATIK AKTUELL BILDVERARBEITUNG FÜR DIE MEDIZIN, Bd. 116, 2004, Seiten 229-233, XP002520018
- SORENSEN ET AL: "Image-guided radiotherapy using a mobile kilovoltage x-ray device" MEDICAL DOSIMETRY, ELSEVIER, US, Bd. 31, Nr. 1, 18. März 2006 (2006-03-18), Seiten 40-50, XP005864688 ISSN: 0958-3947
- YANIV ET AL: "Fluoroscopic image processing for computer aided orthopaedic surgery" MICCAI'98 LNCS, Bd. 1496, 1998, Seiten 325-334, XP002520019

## Beschreibung

Die vorliegende Erfindung betrifft Röntgenmarker und die Zuordnung von Röntgenmarkern zu Bildern dieser Röntgenmarker. Die Bilder der Röntgenmarker werden hierin als Bildmarker bezeichnet.

Bekannte Beispiele für Röntgenmarkervorrichtungen sind Registrierkörper, sogenannte Registrierungskits (engl.: "fluoro registration kits"). Diese sind fest mit dem Bildverstärker eines C-Bogen verbunden. Bei manchen ist eine Drehung relativ zu dem Bildverstärker möglich. An den Registrierungskörpern sind weiter optische Marker (Navigations-Marker) angebracht, die eine Lagebestimmung des Registrierungskörpers mittels eines Navigationssystems erlauben. Die Navigationssysteme werden für die sogenannte bildgestützte Navigation (Image Guided Surgery) eingesetzt. Beispielsweise werden mittels Kameras, die Bestandteile des Navigationssystems sind, die optischen Marker detektiert. Die Auswertung eines Röntgenbildes und der darin abgebildeten Röntgenmarker sowie die bekannte Lage des Registrierkkörpers relativ zum Röntgengerät erlauben die Registrierung eines Instruments in einem gewünschten Bezugssystem, das von dem Navigationssystem vorgegeben werden kann, sowie die virtuelle Darstellung des Instruments im Röntgenbild, ohne dass eine Röntgenaufnahme getätigt wird.

Folgende Druckschriften betreffen derartige Verfahren oder Vorrichtungen:
US 61/054,487
DE 199 17 867 A1

Kann man den Registrierkörper drehen, so gibt es bei der Auswertung des Röntgenbildes, um eine Zuordnung zwischen den Bildmarkern und den Röntgenmarkern zu erzielen, nur die Möglichkeit der Rotation und die Möglichkeit der Spiegelung. Letztere ist gegeben, da das Röntgenbild gespiegelt oder ungespiegelt vom Röntgengerät ausgegeben werden kann. Aufgrund dieser geringen Variationsmöglichkeiten (Rotation und Spiegelung) ist eine Zuordnung offensichtlich. Somit ist eine Zuordnung zwischen den Röntgenmarkern und den Bildmarkern einfach und schnell mittels eines Algorithmus durchführbar.

Für die Zuordnung wird dann z.B. nach den Prinzipien der Lochkamera (Pinhole-Kamera) Informationen über die Röntgenstrahlen- Abbildungsgeometrie bestimmt. Diese Informationen erlauben insbesondere die Lage einer Röntgenquelle relativ zu dem Registrierkörper zu berechnen.
Die Röntgenstrahlen-Abbildungsgeometrie läßt sich z.B. definieren über eine räumliche Transformation ins Projektionszentrum (6 externe Abbildungsparameter: 3 rotatorische, 3 translatorische) sowie 4 interne Abbildungsparameter: 2 Skalierungen, die von Weltkoordinaten [mm] in Pixel des elektronischen Bildes umrechnen sowie die Koordinaten des (rechnerischen) Hauptpunktes ("principal point").

Oben beschriebenes Prinzip gilt grundsätzlich auch für den Scout-View (siehe weiter unten). Bezüglich des Hauptpunktes werden vorzugsweise die beiden Koordinaten (u, v) als der berechnete Mittelpunkt des Bildes aufgefasst.

Die Daten, die obigen Informationen über die Röntgenstrahlen- Abbildungsgeometrie enthalten, werden hierin als Abbildungsgeometrie-Daten bezeichnet. Insbesondere umfassen die Abbildungsgeometrie-Daten die errechnete Projektionsmatrix. Die Abbildungsgeometrie-Daten, und insbesondere die Projektionsmatrix stellen ein allgemeines Abbildungsgesetz für die gegebene Röntgenstrahlen-Abbildungsgeometrie dar. Sie erlauben somit zu berechnen, wie sich ein beliebiger Punkt im Raum im (unverzerrten) Röntgenbild darstellt, wenn man von derselben Röntgenstrahlen-Abbildungsgeometrie ausgeht, wie sie bei der Erstellung des Röntgenbildes gegeben war, das eine Abbildung der Röntgenmarkervorrichtung darstellt. Sie stellt somit eine Verallgemeinerung des Abbildungsvorganges dar, die von dem Spezialfall der Abbildung der Röntgenmarkervorrichtung auf das Röntgenbild bei gegebener Röntgenstrahlen-Abbbildungsgeometrie ausgeht.

Die Berechnung von Abbildungsgeometrie-Daten erfolgt, z.B. mittels Verwendung eines Kameramodells, das die tatsächlichen Abbildungseigenschaften möglichst gut widerspiegelt. Das übliche Kameramodell ist das Lochkamera-Modell. Das heißt die Abbildungsgeometrie-Daten werden z.B. basierend auf den Prinzipien der Lochkamera berechnet. Hierzu wird auf folgende Veröffentlichungen verwiesen, die hiermit durch Bezugnahme in die Offenbarung mit aufgenommen werden:
1. "An Efficient and Accurate Camera Calibration Technique for 3D Machine Vision", Roger Y. Tsai, Proceedings of IEEE Conference on Computer Vision and Pattern Recognition, Miami Beach, FL, 1986, pages 364-374.
2. "A versatile Camera Calibration Technique for High-Accuracy 3D Machine Vision Metrology Using Off-the-Shelf TV Cameras and Lenses", Roger Y. Tsai, IEEE Journal of Robotics and Automation, Vol. RA-3, No. 4, August 1987, pages 323-344, siehe auch unter http://www.cs.emu.edu/~rgw/TsaiDesc.html
3. Veröffentlichung von Ziv Yaniv, "Fluoroscopic X-ray Image Processing and Registration for Computer-Aided Orthopedic Surgery".

Wie man aus dem oben gegebenen Zitat Nr. 2 und der dortigen Internetadresse entnehmen kann, werden in dem dort beschriebenen "Tsai's Camera Model" fünf interne Abbildungsparameter erwähnt, die dort als "internal parameters" bezeichnet werden. Oben wurden jedoch nur vier interne Abbildungsparameter erwähnt. Dies liegt daran, dass im gegebenen Fall von einem unverzerrten Bild ausgegangen wird und deshalb der Parameter "kappa1" nicht erforderlich ist. Falls das Bild verzerrt sein sollte, wäre vorab vorzugsweise ein Entzerrung vorzunehmen (siehe auch Diskussion weiter unten). Allgemein beschreiben die internen Abbildungsparameter, wie die Kamera, also im gegebenen Fall das Röntgengerät ein Bild ausbildet, während die externen Parameter im Weltkoordinatensystem die Lage des Röntgengeräts (Position und Orientierung) beschreiben. Erfindungsgemäß wird das Tsai Kameramodell auf die besonderen Bedingungen der Röntgenstrahlenabbildung angepasst, wie dies inbesondere in der Veröffentlichung von Ziv Yaniv (siehe oben) beschrieben ist. Insbesondere können die beiden Skalierungen f bzw. 1/f und sx verwendet werden.

Sind die Abbildungsgeometrie-Daten bestimmt, so kann durch eine virtuelle Röntgenabbildung des Registrierkörpers überprüft werden, ob die Zuordnung korrekt ist. Die virtuelle Röntgenabbildung wird durch die Abbildungsgeometrie-Daten bestimmt.

Ist die relative Lage zwischen Bildverstärker und Registrierkörper aber unbekannt, so gibt es eine größere Anzahl von möglichen Zuordnungen zwischen den Röntgenmarkern und Bildmarkern, die zu einer erheblichen Rechenzeit von mehreren Minuten oder Stunden (je nach Anzahl der Marker) führen kann. Eine derartige Rechendauer ist in der täglichen Praxis jedoch nicht hinnehmbar. Aufgrund dieser langen Rechenzeit werden Registrierungskörper derzeit in der Praxis fest relativ zum Bildverstärker montiert.

Ein weiteres Beispiel für Röntgenmarkervorrichtungen sind "Röntgenkalibrierphantome" (vgl. DE 102 15 808) oder "Röntgengitter" (vgl. EP 08 156 293 oder US 61/054,487), die zur Bestimmung von Kalibrier-Information zur Kalibrierung einer 3D-CT-Röntgengeräts, insbesondere zur Bestimmung der Lage eines 3D-Röntgen-Messvolumens relativ zu einem vom Navigationssystem vorgegebenen Benutzungssystem genutzt werden können. Die Röntgenmarkervorrichtungen, insbesondere der Träger (z.B. Seitenwände) für die Röntgenmarker und die Röntgenmarker können optisch opak sein.

US 2005/0113682 A1 offenbart ein System zum Konstruieren von Schnittbildern durch ein Auswahlobjekt, wobei identifizierbare Referenzmarker in einer festen Position relativ zum Auswahlobjekt angeordnet sind, die wenigstens zwei identifizierbare Marker umfassen. Dabei wird eine Strahlungsquelle bereitgestellt, um das Auswahlobjekt mit dem Referenzmarker zu bestrahlen. Ein Aufzeichnungsmedium oder Detektor nimmt sodann eine Serie der produzierten Bilder auf.

US 2005/0109855 A1 offenbart ein Navigationssystem für computerunterstützte Chirurgie, das eine Mehrzahl von Markervorrichtungen einsetzt, wobei ein nicht segmentierender gemeinsamer Punkt wenigstens einen Teil dieser Marker miteinander verbindet.

EP 0 853 920 A2 offenbart einen Berechnungsalgorithmus für ein Biopsiesystem, der die Verwendung eines Phantoms mit einer Anzahl von Markern, die symmetrisch um oder asymmetrisch in dem Phantom angeordnet sein können.

Sorensen et al., Image-guided radiotherapy using a mobile kilovoltage x-ray device, Medical Dosimetry, Bd. 31, Nr. 1, Seiten 40 bis 50 offenbart die Verwendung eines Kalibrierphantoms mit pyramidal angeordneten Markern.

DE 197 03 556 A1 offenbart die Verwendung eines Eichphantoms mit einer ersten, Röntgenstrahlung absorbierenden Markeranordnung und mit einer zweiten Markeranordnung für die Kameras, beispielsweise Infrarot-Leuchtdioden bei Verwendung von Infrarot-Kameras.

Schönherr et al., Ein neues algorithmisches Verfahren zur Fluoroskopie-basierten Neuronavigation, Informatik Aktuell - Bildverarbeitung für die Medizin, Band 116, 2004, Seiten 229 bis 233 offenbart einen Algorithmus zur Bestimmung der Lage von Instrumenten in vorher aufgenommen Fluoroskopiebildern, der ein Punktmuster aus zehn Stahlkugeln verarbeitet, wobei jeweils drei Kugeln auf drei zueinander orthogonalen Achsen und eine weitere im Schnittpunkt dieser Achsen liegen.

US 2007/0122020 A1 offenbart eine Anordnung von Röntgenmarkern, die auf Geraden liegen, die Kanten einer Pyramide definieren, wobei eine Strahlenquelle in der Pyramidenspitze liegt.

Aufgabe der Erfindung ist es, eine Zuordnung von Röntgenmarkern zu Bildmarkern auch bei keinerlei Information über die Lage der Röntgenmarkervorrichtung relativ zum Röntgengerät, insbesondere relativ zur Röntgenquelle und zum Bildverstärker zu erleichtern.

Vorstehende Aufgabe wird durch die Gegenstände der unabhängigen Ansprüche gelöst. Die abhängigen Ansprüche sind auf vorteilhafte Weiterbildungen gerichtet.

Eine erfindungsgemäße Röntgenmarkervorrichtung umfasst eine Anordnung von Röntgenmarkern wobei die Anordnung Geraden definiert, die als Vorrichtungs-Geraden bezeichnet werden, und
zumindest ein Teil der Vorrichtungs-Geraden, die als Pyramiden-Geraden bezeichnet werden, Abschnitte aufweisen, die die Kanten von Pyramiden definieren, wobei mindestens eine Spitze jeder der Pyramiden außerhalb eines durch die Röntgenmarker begrenzten Bereichs liegt.

Bei den Geraden und Pyramiden kann es sich insbesondere um virtuelle (geometrische) Gebilde handeln, die durch die physikalischen (realen und materiellen) Röntgenmarker definiert sind.

Vorrichtungs-Geraden, die Abschnitte aufweisen, die Kanten einer Pyramide definieren, werden hierin als "Pyramiden-Geraden" bezeichnet.

Da es sich sowohl bei den Vorrichtungs-Geraden als auch bei den Pyramiden-Geraden um Geraden handelt, sind sie bei einer linearen Abbildung invariant. Beispiele für lineare Abbildungen sind projektive Abbildungen, perspektivische Projektionen, Parallelprojektion und der sogenannte "Scout View" (Mischung aus Parallel- und Perspektivprojektionen. Bei gewöhnlichen Röntgenaufnahmen handelt es sich üblicherweise um perspektivische Projektionen. Bei modernen CT-Scannern ist häufig der vorgenannte Scout View vorherrschend. Letztendlich ist auch eine Parallelprojektion möglich, wenn eine punktförmige Röntgenquelle und der Detektor parallel über den Patienten fahren. Die vorliegende Erfindung nutzt insbesondere die linearen Abbildungseigenschaften der Röntgenbildung. Soweit im Folgenden von einer projektiven Abbildung die Rede ist, ist dies rein beispielhaft. Die erfindungsgemäße Röntgenmarkervorrichtung hat somit eine Anordnung, die eine invariante Eigenschaft aufweist. Dadurch, dass die erfindungsgemäße Anordnung Pyramiden-Geraden definiert, ist diese invariante Eigenschaft im Röntgenbild leichter auffindbar und die Zuordnung zwischen Röntgenmarkern und Bildmarkern wird erleichtert, wie später erläutert wird.

Die vorgenannten Pyramiden-Geraden werden durch Geradenbildungspunkte definiert, die sich wiederum aus der Anordnung der Röntgenmarker ergeben. Nach den Gesetzen der Geometrie benötigt man mindestens zwei Punkte, also Geradenbildungspunkte, um eine Gerade zu definieren. Die Geradenbildungspunkte sind insbesondere durch die Lage der Röntgenmarker, d.h. z.B. durch den jeweiligen geometrischen Mittelpunkt der Röntgenmarker (also z.B. der Mittelpunkt einer Kugel, falls die Röntgenmarker kugelförmig sind) bestimmt. Geradenbildungspunkte können auch Schnittpunkte sein, die sich aus Vorrichtungs-Geraden ergeben, die wiederum durch Geradenbildungspunkte definiert sind, bei denen es sich um Röntgenmarker handelt. Liegen beispielsweise jeweils mindestens zwei Röntgenmarker an den Kanten einer Pyramide, so schneiden sich die Pyramiden-Geraden in einem Vorrichtungs-Schnittpunkt. Dieser Vorrichtungs-Schnittpunkt stellt ebenfalls einen Geradenbildungspunkt dar, der zusammen mit einem anderen Röntgenmarker, der beispielsweise an einer Ecke der Pyramide liegt eine weitere Pyramiden-Gerade bilden kann, die naturgemäß ebenfalls durch dieselbe Pyramidenspitze geht, die aber nicht mit den anderen vorher erwähnten beiden Pyramiden-Geraden identisch ist.

Die Röntgenmarker liegen nur auf einer Seite der Pyramidenspitze aber nicht auf der anderen. Dies gilt, für alle Pyramiden-Geraden. Auf diese Art und Weise kann später die Zuordnung zwischen Röntgenmarkern und Bildmarkern erleichtert werden, da die Nachbarschaftsbeziehungen, also die Reihenfolge ausgehend von dem Vorrichtungs-Schnittpunkt (Pyramidenspitze) genutzt werden kann. Dies wird später noch näher erläutert.

Röntgenmarker sind insbesondere Körper aus einem Material, das die Röntgenstrahlen nicht durchlässt oder zumindest erheblich dämpft. Die Körper können beliebig geformt sein und insbesondere geometrische Grundformen, wie Kugel oder Quader einnehmen. Die Körper sind insbesondere voneinander beabstandet. Der Abstand ist insbesondere größer als der Körperdurchmesser der Röntgenmarker. Körperdurchmesser sind vorzugsweise größer als 1mm und/oder kleiner als 3cm.

Auf den Pyramiden-Geraden befinden sich jeweils mindestens zwei Röntgenmarker, um Artefakte später im Röntgenbild besser identifizieren zu können. Die Pyramidenspitze befindet sich außerhalb des Bereichs, in der sich die Röntgenmarker befinden. In diesem Fall liegt kein Röntgenmarker auf der Pyramidenspitze.

Die vorgenannten Geraden, insbesondere die Vorrichtungs-Geraden und Pyramiden-Geraden sind bei einer projektiven Abbildung invariant. Die erfindungsgemäße Anordnung der Röntgenmarker beschreibt somit eine invariante Eigenschaft.

Invariante Eigenschaften sind Eigenschaften, die bei einer projektiven Abbildung erhalten bleiben. Insbesondere sind diese Eigenschaften Invarianten einer jeden projektiven Abbildung. Insbesondere stellen sie ein kennzeichnendes Merkmal der projektiven Geometrie dar. Invariante Eigenschaften der projektiven Geometrie sind beispielsweise Geraden, die durch die Anordnung der Röntgenmarker definiert werden, wie z.B. die Vorrichtungs-Geraden, und die hierin auch als "invariante Geraden" bezeichnet werden. Ein weiteres Beispiel ist das sogenannte Doppelverhältnis oder im englischen "cross ratio". Dieses Doppelverhältnis stellt ebenfalls eine invariante Eigenschaft dar, stellt also eine invariante der projektiven Abbildung dar. Das Doppelverhältnis ist in der Geometrie eine Zahl, die die gegenseitige Lage von vier verschiedenen auf einer Geraden gelegenen Punkten kennzeichnet. Zwei dieser Punkte bestimmten dabei eine Strecke, die von den beiden anderen Punkten geteilt wird. Das Doppelverhältnis wird definiert als das Verhältnis der beiden Teilverhältnisse.

Die Röntgenmarkervorrichtung definiert insbesondere mindestens 6 Geradenbildungspunkte. Diese mindestens 6 Geradenbildungspunkte werden vorzugsweise aus Röntgenmarkern bestimmt. Insbesondere eine der mindestens 6 Geradenbildungspunkte kann auch ein Vorrichtungs-Schnittpunkt, insbesondere eine Pyramidenspitze sein, bei dem bzw. bei der sich kein Röntgenmarker befindet. Die mindestens 6 Geradenbildungspunkte liegen vorzugsweise nicht alle in einer Ebene.

Insbesondere ist die Gesamtzahl der Röntgenmarker kleiner 30 oder 20 oder 10. Insbesondere ist der Maximalabstand zwischen zwei beliebigen Röntgenmarkern der Röntgenmarkervorrichtung kleiner als der Durchmesser eines Detektionsbereiches eines Röntgengeräts, insbesondere kleiner 50 cm oder 30 cm oder 20 cm oder 10 cm..

Vorzugsweise liegen mindestens 6 der Röntgenmarker auf invarianten Geraden. Sie werden hierin auch als invariante Röntgenmarker bezeichnet, da sie eine invariante Eigenschaft definieren.

Vorzugsweise enthalten mindestens drei der Pyramiden-Geraden jeweils einen Abschnitt, der jeweils einer Seitenkante ein und derselben Pyramide entspricht. Vorzugsweise haben mindestens drei der Pyramiden-Geraden Abschnitte, die jeweils sowohl eine Seitenkante einer Pyramide als auch der Grundkante mindestens einer anderen Pyramide, insbesondere den Grundkanten zweier anderer Pyramiden entsprechen. Vorzugsweise liegen alle Röntgenmarker auf Pyramiden-Geraden.

Wie erwähnt, kann es sich bei den Geradenbildungspunkten auch um Vorrichtungs-Schnittpunkte handeln. Vorrichtungs-Schnittpunkte erfüllen zumindest die Bedingung, dass sich in ihnen mindestens zwei Vorrichtungs-Geraden schneiden. Ein Vorrichtungs-Schnittpunkt kann mit der Lage eines Röntgenmarkers zusammenfallen oder er kann sich auch an einer Stelle im Raum ergeben, an der sich kein Röntgenmarker befindet, an dem sich aber dennoch mindestens zwei Vorrichtungs-Geraden schneiden. Vorzugsweise ist ein Schnittpunkt nur dann ein Vorrichtungs-Schnittpunkt, wenn sich in ihm eine bestimmte vorgegebene Anzahl von Vorrichtungs-Geraden schneidet und/oder die Anzahl größer oder gleich einer vorgegebenen Mindestanzahl (z.B. 3, 4 oder 5) ist und/oder kleiner oder gleich einer vorgegebenen Maximalanzahl (z.B. Gesamtanzahl der Röntgenmarker der Röntgenmarkervorrichtung minus 1 oder minus 2). Die vorgegebene Anzahl ist vorzugsweise mindestens drei. Die vorgegebene Anzahl kann für alle Vorrichtungs-Schnittpunkte gleich sein oder sie können verschieden sein. Durch die vorgegebene Anzahl, Mindestanzahl oder Maximalanzahl lassen sich Artefakte leichter identifizieren.

Vorzugsweise liegen mindestens 6 der Röntgenmarker auf Vorrichtungs-Geraden, sind also insbesondere Geradenbildungspunkte. Vorzugsweise liegen mindestens drei Geradenbildungspunkte auf den Vorrichtungs-Geraden, insbesondere Pyramiden-Geraden.

Insbesondere stimmt somit die Lage einer jeden der Geradenbildungspunkte entweder mit der Lage eines der Röntgenmarker (insbesondere der Lage des geometrischen Mittelpunktes des Röntgenmarkers) oder der Lage einer der Vorrichtungs-Schnittpunkte überein.

Die durch die Anordnung definierten Vorrichtungs-Geraden, insbesondere die Pyramiden-Geraden, liegen vorzugsweise nich alle in einer Ebene, sondern insbesondere in mindestens zwei Ebenen.

Durch die erfindungsgemäße Anordnung wird eine Zuordnung zwischen den Röntgenmarkern und den Bildmarkern in einem Röntgenbild wesentlich erleichtert. Dadurch, dass die Röntgenmarker auf Vorrichtungs-Geraden liegen, die bei der Abbildung invariant sind, kann in dem Röntgenbild nach entsprechenden Geraden gesucht werden (beispielsweise mittels eines Algorithmus), die durch Bildmarkerkandidaten (also möglichen Bildmarkern) gebildet werden. Dadurch, dass vorzugsweise mindestens 6 Geradenbildungspunkte, insbesondere mindestens 6 Röntgenmarker auf Vorrichtungs-Geraden liegen, können diese für einen Algorithmus, der auch die Lochkamera-Prinzipien benutzt (siehe oben), und der die Abbildungsgeometrie-Daten, die insbesondere die Geometrie der projektiven Abbildung beschreiben, berechnet, verwendet werden. Dadurch, dass die Röntgenmarker auf den Vorrichtungs-Geraden liegen, kann für sie leichter die korrekte Zuordnung gefunden werden.

Die Zuordnung wird weiter dadurch erleichtert, dass vorzugsweise eine Mindestanzahl von drei Geradenbildungspunkten, die größer als zwei ist (beispielsweise 3 oder 4 oder 5), auf zumindest einem Teil der Vorrichtungs-Geraden, insbesondere der Pyramiden-Geraden liegen. Dies bedeutet, dass auch im Röntgenbild bei einer fehlerfreien Abbildung ebenfalls die Mindestanzahl von Geradenbildungspunkten im Röntgenbild identifizierbar sein müssten. Dies verringert den Einfluss von Artefakten auf die Auswertung des Röntgenbildes, um Bilder der Röntgenmarker (sogenannte Bildmarker) zu erkennen. Liegt ein Bildmarkerkandidat im Röntgenbild und wird dieser über eine Gerade mit einem Bildmarker oder einem anderen Bildmarkerkandidaten verbunden, so müsste diese Gerade durch einen weiteren Geradenbildungspunkt verlaufen, falls die Mindestanzahl von Geradenbildungspunkten größer zwei ist, um zu bestätigen, dass es sich bei dem Bildmarkerkandidaten um einen tatsächlichen Bildmarker handelt. Ist dies nicht der Fall, so kann der Bildmarkerkandidat als wahrscheinliches Artefakt von dem Zuordnungsalgorithmus ausgeschlossen werden. Letzteresinsbesondere dann, wenn alle Röntgenmarker auf Vorrichtungs-Geraden liegen, die durch eine Mindestanzahl von Geradenbildungspunkten, die größer zwei ist, definiert sind.

Gemäß einer Ausführungsform liegen alle Röntgenmarker auf Pyramiden-Geraden. Die Pyramiden-Geraden schneiden sich in mindestens einer Pyramidenspitze, wobei die Anzahl der sich schneidenden Pyramiden-Geraden bekannt ist. Verbindet man die Bildmarkerkandidaten in einem Röntgenbild, lassen sich somit die Bildmarker identifizieren, indem man nur diejenigen Bildmarkerkandidaten auswählt, die auf Bild-Geraden liegen, die sich in einem Bild-Schnittpunkt mit einer vorbestimmten Eigenschaft schneiden. Die vorbestimmte Eigenschaft liegt darin, dass sich in dem Bild-Schnittpunkt eine Anzahl von Bild-Geraden schneidet, die der Anzahl der Pyramiden-Geraden entspricht, die sich in zumindest einer Pyramidenspitze schneiden. Durch die Röntgenmarkervorrichtung ist die Anzahl der sich in einer Pyramidenspitze schneidenden Pyramiden-Geraden vorgegeben. Dadurch ist auch die Bedingung bekannt, die ein Bild-Schnittpunkt erfüllen muss, durch den die Bild-Geraden laufen, auf denen die Bildmarker liegen.

Die Anzahl der Geradenbildungspunkte, insbesondere Röntgenmarker, kann für alle Vorrichtungs-Geraden gleich sein. Dies kann das Erkennen von Artefakten erleichtern. Sie kann aber auch für bestimmte Geraden anders sein und sich insbesondere um zwei oder mehr Geradenbildungspunkte (insbesondere Röntgenmarker) von den anderen Vorrichtungs-Geraden unterscheiden, um die Identifikation und damit Zuordnung der Geraden zu erleichtern. Andererseits ist das Risiko einer gegenseitigen Abdeckung höher, je dichter die Röntgenmarker auf den Vorrichtungs-Geraden liegen.

Da Geraden unter Projektionen invariant sind, wird bevorzugt angenommen, dass auch die Anzahl der Geradenbildungspunkte invariant ist. Vorzugsweise wird die Röntgenmarkervorrichtung so gestaltet, dass auf den Vorrichtungs-Geraden, insbesondere auf den Pyramiden-Geraden eine Mindestanzahl von Geradenbildungspunkten und/oder eine Mindestanzahl von Röntgenmarkern liegen. Gehen Bild-Geraden aus einer Abbildung von Vorrichtungs-Geraden, insbesondere Pyramiden-Geraden hervor, so müssen sie die entsprechende Bedingung erfüllen. Es muss also eine Mindestanzahl von Geradenbildungspunkt-Kandidaten oder eine Mindestanzahl von Bildmarkerkandidaten auf der Bild-Geraden geben. Geradenbildungspunkte in einem Röntgenbild sind Bildmarker oder Schnittpunkte, die Vorrichtungs-Schnittpunkten, insbesondere Pyramidenspitzen entsprechen. Aufgrund der vorgenannten bekannten Mindestanzahl lassen sich somit Artefakte mit einer hohen Wahrscheinlichkeit ausschließen.

Dies reduziert die Anzahl der möglichen Zuordnungen in der Praxis erheblich und damit auch die Rechenzeit. Zusammengefasst gilt: Je größer die Mindestanzahl der Geradenbildungspunkte und/oder Röntgenmarker mit einer Vorrichtungs-Geraden, insbesondere Pyramiden-Geraden, desto sicherer können scheinbare Bildmarker (Artefakte) identifiziert werden.

Die Rechenzeit kann weiter dadurch verringert werden, dass die Röntgenmarker nach Vorrichtungs-Geraden, insbesondere Pyramiden-Geraden gruppiert werden und in dem Röntgenbild ebenfalls Geraden identifiziert werden, die Eigenschaften haben, die den Eigenschaften der Vorrichtung-Geraden, insbesondere Pyramiden-Geraden entsprechen. Diese Geraden werden hierin als Bild-Geraden bezeichnet. Die Bild-Geraden umfassen mindestens einen Bildmarker und werden vorzugsweise ebenfalls durch eine Mindestanzahl von Geradenbildungspunkten definiert, die größer als zwei ist (beispielsweise 3, 4 oder 5). Geradenbildungspunkte für Bild-Geraden haben die entsprechenden Eigenschaften wie Geradenbildungspunkte für Vorrichtungs-Geraden. "Entsprechend" bedeutet hierin, dass Bildmarker den Röntgenmarkern entsprechen und "Bild-Schnittpunkte" den Vorrichtungs-Schnittpunkten, insbesondere den Pyramidenspitzen entsprechen. Sind im Falle einer Vorrichtungs-Geraden mindestens zwei der Geradenbildungspunkte Röntgenmarker, so sind entsprechend im Fall der Bild-Geraden mindestens zwei der Geradenbildungspunkte Bildmarker. Weiter können Bild-Schnittpunkte Geradenbildungspunkte für Bild-Geraden darstellen. Bild-Schnittpunkte haben dabei dieselben Eigenschaften, wie die Vorrichtungs-Schnittpunkte, wobei die Bild-Geraden den Vorrichtungs-Geraden entsprechen. Erfüllen beispielsweise Vorrichtungs-Schnittpunkte, insbesondere Pyramidenspitzen die Bedingung, dass sich in ihnen eine bestimmte Anzahl von Vorrichtungs-Geraden, insbesondere Pyramiden-Geraden schneiden, so erfüllen die entsprechenden Bild-Schnittpunkte die Bedingung, dass sich in ihnen dieselbe Anzahl von Bild-Geraden schneidet, falls keine Artefakte stören. Ist eine Mindestanzahl und/oder Maximalanzahl für die Anzahl der Vorrichtungs-Geraden (insbesondere Pyramiden-Geraden), die sich in den Vorrichtungs-Schnittpunkten (insbesondere Pyramidenspitzen) schneiden vorgegeben, so erfüllen die Bild-Schnittpunkte die entsprechende Bedingung, falls keine Artefakte stören. In den Bild-Schnittpunkten sollte somit die Zahl der sich schneidenden Bild-Geraden größer oder gleich der Mindestanzahl und/oder kleiner oder gleich der Maximalanzahl sein. Werden Abweichungen festgestellt, so kann dies als Hinweis darauf gewertet werden, dass es sich bei einem Schnittpunkt im Bild um keinen Bild-Schnittpunkt handelt (oder dass Artefakte diese Abweichung verursachen).

Gemäß einer Ausführungsform werden diejenigen Geraden im Bild als Bild-Geraden ausgewählt, die die Eigenschaften der Pyramiden-Geraden erfüllen. Sind für die Pyramidenspitzen eine Mindestanzahl und/oder eine Maximalanzahl vorgegeben für die Anzahl der sich in der Pyramidenspitze schneidenden Pyramiden-Geraden, so werden die Schnittpunkte im Bild darauf überprüft, ob sich in ihnen die bekannte Mindestanzahl und/oder Maximalanzahl an Geraden schneiden. Nur wenn sie diese Bedingung erfüllen, werden sie als Bild-Geraden, die Pyramiden-Geraden entsprechen, für weitere Verfahrensschritte ausgewählt. Entsprechend werden nur dann die Schnittpunkte als Bild-Schnittpunkte für weitere Verfahrensschritte ausgewählt, wenn sie Pyramidenspitzen entsprechen.

Die vorgenannte Mindestanzahl und Maximalanzahl kann durch die Röntgenmarkervorrichtung vorgegeben sein. So kann die Mindestanzahl der Mindestanzahl an Pyramiden-Geraden entsprechen, die sich in einer der Pyramidenspitzen schneiden und die Maximalanzahl kann der Maximalanzahl der Pyramiden-Geraden entsprechen, die sich in einer der Pyramidenspitzen schneiden. Die Anzahl der sich jeweils in den Pyramidenspitzen schneidenden Geraden kann insbesondere für alle Pyramidenspitzen gleich sein. In diesem Fall gibt es z.B. nur eine einzige vorgegebene Anzahl. Beim erfindungsgemäßen Verfahren kann aber die vorgenannte Mindestanzahl oder Maximalanzahl auch dazu verwendet werden, um Artefakte möglichst auszuschließen. In einem Röntgenbild ergeben sich durch Verbindung der Bildmarker oder Bildmarkerkandidaten eine Vielzahl zufälliger Schnittpunkte, in denen sich typischerweise aber nur zwei Geraden schneiden. Setzt man die Mindestanzahl zur Auswahl der Bild-Schnittpunkten größer als zwei (insbesondere größer als drei), so kann man damit eine fehlerhafte Auswahl eines Bild-Schnittpunktes mit einer hohen Wahrscheinlichkeit vermeiden. Um Bild-Schnittpunkte, die Pyramidenspitzen entsprechen, nicht mit Bildmarkern zu verwechseln, die nicht auf einer Pyramidenspitze liegen, in denen sich aber mehrere Geraden schneiden, kann man auch eine Maximalanzahl festlegen. Diese Maximalanzahl ist insbesondere kleiner als die Zahl der Röntgenmarker minus 1 und entspricht beispielsweise der maximalen Anzahl von Pyramiden-Geraden, die sich in einer Pyramidenspitze schneiden oder liegt zwischen dieser maximalen Anzahl und der Anzahl der Röntgenmarker minus 1. Der Grund für die Maximalanzahl, die bei der Identifikation verwendet wird, liegt darin, dass sich in Bild-Schnittpunkten, die Pyramidenspitzen entsprechen, typischerweise weniger Geraden schneiden, als in Bild-Schnittpunkten, die mit Bildmarkern übereinstimmen, die Röntgenmarkern entsprechen, die nicht auf einer Pyramidenspitze liegen.

Bei dem erfindungsgemäßen Verfahren kann die Mindestanzahl und/oder Maximalanzahl also genutzt werden, indem ein Schnittpunkt nur dann als Bild-Schnittpunkt ausgewählt wird, falls er folgende Bedingung erfüllt: Anzahl der sich schneidenden Bild-Geraden größer oder gleich (Mindestanzahl - X) und/oder kleiner oder gleich (Maximalzahl + Y), wobei die Mindestanzahl der geringsten Anzahl, der sich in einer Pyramidenspitze schneidenden Pyramiden-Geraden entspricht und die Maximalanzahl der größten Anzahl der sich in einer Pyramidenspitze schneidenden Geraden entspricht und wobei X und Y ganze Zahlen größer oder gleich Null sind. X>0 und Y>0 erlauben somit Artefakte in einem gewissen Umfang zu tolerieren und somit das Bild trotzdem auszuwerten, wenn zufällig Artefakte eine weitere Bildgerade generieren, die durch einen Bild-Schnittpunkt geht, oder wenn Bildmarker im Bild fehlen, so dass eine Bild-Gerade im Bild-Schnittpunkt fehlt.

Schneiden sich, falls die Mindestanzahl größer 2 ist, in einem Schnittpunkt im Bild mehr als zwei Bild-Geraden, so ist dies bereits ein Hinweis darauf, dass es sich um einen Bild-Schnittpunkt handelt, da sich typischerweise an Schnittpunkten, an denen sich keine Bildmarker befinden, nur zwei Geraden schneiden. Gemäß einer Ausführungsform kann man somit Bild-Schnittpunkten insbesondere die Bedingung auferlegen, dass sich in ihnen eine Mindestanzahl von Bild-Geraden schneiden, wobei die Mindestanzahl mindestens drei beträgt.

Die Bildmarker können nach Bild-Geraden gruppiert werden, die insbesondere wie oben beschrieben, im Bild ausgewählt wurden, die also Vorrichtungs-Geraden und insbesondere Pyramiden Geraden entsprechen. Die Bestimmung der Zuordnungsmöglichkeiten kann dann gruppenweise zwischen Röntgenmarkern, die auf Vorrichtungs-Geraden (insbesondere Pyramiden-Geraden) liegen und Bildmarkern, die auf Bild-Geraden liegen, erfolgen. Dies verringert die erforderliche Rechenleistung erheblich. Dies wird weiter unten im Zusammenhang mit der Beschreibung des erfindungsgemäßen Verfahrens weiter erläutert.

Die Anordnung der Röntgenmarker ist dergestalt, dass sie mindestens sechs Vorrichtungs-Geraden definieren die sich in zwei Vorrichtungs-Schnittpunkten schneiden. Für drei oder mehr Vorrichtungs-Geraden gibt es also einen gemeinsamen Vorrichtungs-Schnittpunkt, so dass sich z.B. drei Vorrichtungs-Geraden oder vier Vorrichtungs-Geraden in dem gemeinsamen Vorrichtungs-Schnittpunkt schneiden.

Vorzugsweise liegt mindestens ein Teil der Röntgenmarker, vorzugsweise mindestens 6 an den Ecken von mindestens einem Pyramidenstumpf. Der Pyramidenstumpf hat vorzugsweise mindestens drei Seitenflächen, vorzugsweise 4 oder 5 Seitenflächen, oder mehr. Beispielsweise liegt die Anzahl der Seitenflächen des Pyramidenstumpfes zwischen drei und zehn, z.B. zwischen drei und sechs. Beispielsweise beträgt die Anzahl der Seitenflächen 4. Letzteres bedeutet, dass der Pyramidenstumpf der Geometrie eines Würfels ähnlich ist, aber nicht gleich ist. Vorzugsweise liegt zumindest ein Teil der Röntgenmarker an den Ecken des Pyramidenstumpfes und somit auf Pyramiden-Geraden.

Gemäß der vorliegenden Erfindung definieren die Röntgenmarker somit Anordnungen, die eine Pyramidenstruktur, wie z.B. einen Pyramidenstumpf umfassen. Die Pyramidenspitze ist ein Geradenbildungspunkt, auf dem sich kein Röntgenmarker befindet.

Vorzugsweise schneiden sich die Vorrichtungs-Geraden im Vorrichtungs-Schnittpunkt in einem Winkel ungleich 90°, um das Risiko von Überdeckungen im Röntgenbild zu verringern. Der Winkel kann stumpf sein, ist aber vorzugsweise spitz, um unabhängig von der Blickrichtung auf die Röntgenmarkervorrichtung die Variation des Abstandes zwischen dem nächstliegenden und dem am weitesten weg liegenden Röntgenmarker, die in einem vorgegebenen Volumen liegen, das von Röntgenstrahlen durchstrahlt werden soll, möglichst gering zu halten. Der spitze Winkel ist kleiner 90° und vorzugsweise kleiner 80°, 70°, 60°, 50°, 40° oder 30°. Die Röntgenmarker können insbesondere und vorzugsweise an den Ecken und/oder Kanten des Pyramidenstumpfes liegen, aber auch auf den Seiten- und/oder Grundflächen der Pyramide.

Die Pyramidenspitzen liegen außerhalb eines Bereichs, der durch Röntgenmarker begrenzt ist. Dies gilt vorzugsweise zumindest für einen Großteil der Vorrichtungs-Schnittpunkte, insbesondere Pyramidenspitzen. Dies hat den Vorteil, dass zur Bestimmung der Zuordnungsmöglichkeiten auch Punkte außerhalb des Röntgenmarker-Bereichs genutzt werden können. Röntgenmarker liegen vorzugsweise innerhalb des Strahlungsbereichs des Röntgengeräts, der auf das Röntgenbild abgebildet wird, um bei der Bestimmung der Zuordnungen mitwirken zu können. Die Vorrichtungs-Schnittpunkte müssen jedoch nicht notwendigerweise innerhalb dieses Strahlungsbereichs liegen, falls sie virtuell sind. Somit kann durch die Anordnung der Vorrichtungs-Schnittpunkte außerhalb des Röntgenmarker-Bereichs die Anzahl der Punkte, für die eine Zuordnungsmöglichkeit gefunden werden kann, vergrößert werden, ohne die Röntgenmarker dichter zu packen. Ein dichteres Packen der Röntgenmarker würde das Risiko der gegenseitigen Überdeckung erhöhen, was die Auswertung des Röntgenbildes erschwert. Andererseits erleichtert eine höhere Anzahl von Geradenbildungspunkten das Ausschließen und Erkennen von Artefakten.

Die Röntgenmarkervorrichtung kann zum Registrieren und zum Kalibrieren eingesetzt werden. Gemäß einer bevorzugten Ausführungsform ist die Röntgenmarkervorrichtung Bestandteil eines Registrierkörpers, der die Registrierung eines Gegenstands in einem Bezugssystem eines Navigationssystems erlaubt. Die Registrierung erfolgt insbesondere in einem Bezugssystem, in dem das Röntgenbild ruht und/oder in dem eine im Röntgenbild abgebildete Körperstruktur eines Patienten ruht.

Gemäß einer anderen Ausführungsform kann die Röntgenmarkervorrichtung auch dazu verwendet werden, Kalibrier-Information für ein Röntgengerät zu bestimmen. Insbesondere kann die erfindungsgemäße Röntgenmarkervorrichtung erfindungsgemäß verwendet werden, um die genannte Kalibrier-Information zu bestimmen. Diese Kalibrier-Information umfasst Information über die Lagebeziehung und/oder räumliche Beziehung zwischen einem Bezugssystem, in dem das Röntgengerät ruht und, im Falle eines dreidimensionalen Röntgenscans, einem 3D-Scan-Bezugssystem, in dem ein aus dem 3D-Röntgenscandaten ermitteltes Modell eines Körpers ruht (siehe EP 08 156 293.6).

Vorzugsweise umfasst die erfindungsgemäße Röntgenmarkervorrichtung eine Navigations-Markervorrichtung und ist insbesondere als Registrierkörper ausgebildet. Eine Navigations-Markervorrichtung umfasst eine Anordnung von Navigations-Markern. Navigations-Marker sind Marker, die von der Detektionseinrichtung eines Navigationssystems detektiert werden können. Es kann sich bei Navigations-Markern um aktive oder um passive Marker handeln, die Strahlen und/oder Wellen aussenden oder reflektieren. Bei den Strahlen oder Wellen kann es sich um elektromagnetische Strahlen oder Wellen, wie beispielsweise Licht oder Infrarotlicht handeln. Auch kann es sich beispielsweise um Schallwellen, wie Ultraschallwellen handeln. Durch die Detektion der Navigations-Marker kann das Navigationssystem die Lage der Navigations-Marker bestimmen. Da die relative Lage zwischen Navigations-Markern vorzugsweise bekannt ist, lässt sich die Navigations-Markervorrichtung identifizieren. Auch ist vorzugsweise die relative Lage zwischen den Navigations-Markern und den Röntgenmarkern bekannt, so dass aus der detektierten Lage der Navigations-Marker auf die Lage der Röntgenmarker geschlossen werden kann. Durch daserfindungsgemäße Verfahren kann die relative Lage der Röntgen-Abbildungsgeometrie relativ zu den Röntgenmarkern bestimmt werden. Somit ist auch dem Navigationssystem die Lage der Röntgen-Abbildungsgeometrie bekannt, wenn die Navigations-Marker detektiert werden. Wird nun ein Gegenstand, beispielsweise Instrument oder Implantat verwendet, an dem ebenfalls Navigations-Marker angebracht sind, deren Lage relativ zu dem Gegenstand bekannt sind, kann das Navigationssystem die Lage des Gegenstands relativ zu der Röntgen-Abbildungsgeometrie berechnen. Insbesondere kann das System (mit Hilfe der Abbildungsgeometrie-Daten) berechnen, welche Lage der Gegenstand (z.B. das Instrument) im Röntgenbild einnehmen würde, wenn er von Röntgenstrahlen durchstrahlt werden würde, die den durch die Röntgen-Abbildungsgeometrie vorgegebenen Bedingungen folgen. Somit kann ohne Betätigung oder Einsatz der Röntgeneinrichtung virtuell errechnet werden, welche Lage der Gegenstand im Röntgenbild einnimmt. Die Navigations-Marker sind vorzugsweise von den Röntgenmarkern beabstandet angeordnet. Vorzugsweise ist der Mindestabstand zwischen einem (beliebigen) der Navigations-Marker und einem (beliebigen) der Röntgenmarker größer als der Mindestabstand zwischen den Röntgenmarkern untereinander, insbesondere auch größer als der Maximalabstand zwischen den Röntgenmarkern.

Gemäß einer Ausführungsform wird dem Navigationssystem mitgeteilt, wo zumindest in etwa der Bildverstärker, also die Bildebene des Röntgengeräts liegt. Dies kann man beispielsweise durch Anbringen von Navigations-Markern am Röntgengerät, insbesondere am Bildverstärker und/oder an der Röntgenquelle bewirken. Auch kann mittels eines Pointers die Richtung von Röntgenquelle zur Bildverstärker zu einem Bediener für das Navigationssystem erkennbar angegeben werden. Die Detektion der Navigations-Marker der Röntgenmarkervorrichtung kann dann genutzt werden, um zumindest grob die Lage der Röntgenmarkervorrichtung relativ zu Quelle und Bildverstärker zu bestimmen. Diese grob bestimmte Lage kann dann genutzt werden, um eine intelligente Wahl für eine anfängliche Zuordnung zu Beginn des Zuordnungsverfahrens zu nutzen. Insbesondere kann so bestimmt werden, welche Bild-Schnittpunkte welchen Pyramidenspitzen mit der größten Wahrscheinlichkeit entsprechen. Mit einer derartig bestimmten, wahrscheinlichen Zuordnung zwischen Bild-Schnittpunkten und Pyramidenspitzen kann dann das Zuordnungsverfahren begonnen werden. Somit kann die Wahrscheinlichkeit erhöht werden, dass das Zuordnungsverfahren schneller die korrekte Zuordnung findet.

Die erfindungsgemäße Röntgenmarkervorrichtung ist vorzugsweise (in alle Richtungen) frei beweglich relativ zu dem Röntgengerät, also insbesondere nicht ortsfest relativ zu dem Röntgengerät angebracht oder fixiert und ohne Einschränkung von Freiheitsgraden. Insbesondere muss sie vorzugsweise nicht am Röntgengerät angebracht oder fixiert werden. Dies erleichtert ihre Handhabung. Insbesondere wird dadurch der Zeitaufwand gegenüber herkömmlichen Registrierungskits verringert. Außerdem lässt sich die erfindungsgemäße Röntgenmarkervorrichtung, da sie vorzugsweise frei beweglich ist und nicht fixiert ist, leichter sterilisieren. Auch wird die Mechanik des Röntgengeräts durch die erfindungsgemäße Röntgenmarkervorrichtung, die vorzugsweise nicht am Röntgengerät fixiert ist, nicht belastet. Vorteilhaft müssen keine sterilen Abdeckungen um die erfindungsgemäße Röntgenmarkervorrichtung gelegt werden, da eine vollständige Sterilisierung aufgrund der freien Bewegbarkeit möglich ist. Insbesondere wird somit nicht durch eine sterile Abdeckung die Qualität des Röntgenbilds beeinträchtigt.

Die vorliegende Erfindung hilft vorteilhaft insbesondere bei den folgenden Herausforderungen: zum Ersten werden vorzugsweise so viele Bildmarker wie möglich im Röntgenbild identifiziert. Dabei wird insbesondere davon ausgegangen, dass die Marker eines Entzerrgitters, sofern vorhanden, bereits entfernt wurden. Zum Zweiten werden vorzugsweise Artefakte erkannt, d.h. es sollte vorzugsweise korrekt erkannt werden, dass ein Bildmarkerkandidat ein Artefakt oder ein Bildmarker ist. Zum Dritten werden vorzugsweise die identifizierten Bildmarker in eine korrekt Korrelation (Zuordnung) zu den physikalischen Markern, also den Röntgenmarkern gebracht. Die erfindungsgemäße Nutzung der invarianten Eigenschaften hilft bei der Lösung aller drei vorgenannten Herausforderungen. Insbesondere der zweite und dritte Punkt können miteinander verwoben sein.

Die vorliegende Erfindung betrifft weiterhin ein Verfahren zum Bestimmen einer Zuordnung zwischen Röntgenmarkern der Röntgenmarkervorrichtung und Bildmarker eines Röntgenbildes. Die Erfindung betrifft die Verwendung der erfindungsgemäßen Röntgenmarkervorrichtung bei dem erfindungsgemäßen Verfahren.

Ist die korrekte Zuordnung gefunden, so ist auch die der Zuordnung entsprechende Röntgenstrahlen-Abbildungsgeometrie bestimmt, die der Abbildung der Röntgenmarkervorrichtung zur Erzeugung des Röntgenbildes zugrunde liegt und die durch Abbildungsgeometrie-Daten beschrieben wird. Man geht bei der Röntgenabbildung davon aus, dass es sich um eine projektive Abbildung handelt, die insbesondere den Gesetzen folgt, die den Prinzipien der Lochkamera zugrunde liegen.

Basierend auf einer bekannten Röntgenstrahlen-Abbildungsgeometrie ist es insbesondere möglich, die Lage einer Röntgenquelle relativ zu der Röntgenmarkervorrichtung zu berechnen. Die errechnete Lage der Röntgenquelle entspricht anschaulich dem Schnittpunkt in dem sich rückwärts laufende Röntgenstrahlen, die das Röntgenbild erzeugen, schneiden würden. Ist die Projektionsmatrix bekannt, so kann basierend hierauf, falls beispielsweise die Lage eines Gegenstands relativ zu dieser errechneten Lage bekannt ist, errechnet werden, welches Bild dieser Gegenstand auf einem von der Röntgenquelle erzeugten Röntgenbild ergeben würde. Die Projektionsmatrix ist ein Beispiel für eine Abbildungsmatrix, die einen dreidimensionalen Punkt im Raum (z.B. in einem Weltkoordinatensystem) auf einen zweidimensionalen Punkt abbildet, der in einer Ebene liegt, für die eine Detektion der Strahlen angenommen wird. Die Abbildungsmatrix beschreibt also die Abbildung der Röntgenmarker auf das Röntgenbild. Allgemein hat die Abbildungsmatrix 11 Freiheitsgrade. Nimmt man als vereinfachtes Kameramodell zur Beschreibung der Abbildung ein Lochkameramodell an, so lassen sich die Freiheitsgrade auf 10 reduzieren.
Beide Matrizen (mit 10 oder mit 11 Parametern) sind Projektionsmatrizen. Der Unterschied bei der 10-Parameter-Version ist, dass hier insbesondere die Voraussetzung eingeht, daß die Achsen des Bildsensors orthogonal zueinander sind. Der allgemeinere Fall mit 11 Parametern erlaubt eine Scherung zwischen diesen beiden Achsen.

Bei dem erfindungsgemäßen Verfahren ist die Anordnung von Röntgenmarkern im Raum bekannt. Hierbei kann es sich um die Anordnung aller Röntgenmarker der Röntgenmarkervorrichtung handeln oder um einen Teil dieser Röntgenmarker. Vorzugsweise ist zumindest die Anordnung derjenigen Röntgenmarker bekannt, die invariante Eigenschaften definieren, die bei einer projektiven Abbildung erhalten bleiben. Vorzugsweise umfasst die bekannte Anordnung zumindest 5, vorzugsweise wenigstens 6 der Röntgenmarker, die insbesondere auf mindestens zwei Ebenen liegen und mindestens zwei Geraden definieren. Die Röntgenmarkervorrichtung erfüllt die Eigenschaften der bereits oben erwähnten erfindungsgemäßen Röntgenmarkervorrichtung.

Ziel des erfindungsgemäßen Verfahrens ist es, die Zuordnung zwischen Röntgenmarkern und Bildmarkern zu bestimmen, um so eine Voraussetzung für die Bestimmung der Röntgenstrahlen-Abbildungsgeometrie zu erhalten. Somit ist bei der Durchführung des Verfahrens die Röntgenstrahlen-Abbildungsgeometrie unbekannt oder nicht vollständig bekannt. Bezüglich der Abbildungsgeometrie ist bekannt, dass sie den Bedingungen der linearen Abbildung, insbesondere der projektiven Abbildung genügt. (Dabei wird insbesondere vorausgesetzt, dass das Bild keine Verzerrungen durch den Bildverstärker aufweist. Das ist der Fall für C-Bögen mit Flach-Panel-Detektoren. Im Falle von konventionellen Bildverstärkern ist vorzugsweise noch ein Verarbeitungsschritt der Bildentzerrung vorgeschaltet.) Die Informationen sind jedoch nicht ausreichend, um die relative Lage der Röntgenstrahlenquelle (insbesondere der errechneten Röntgenstrahlenquelle) relativ zu der Röntgenmarkervorrichtung, insbesondere relativ zu der Anordnung der Röntgenmarker zu bestimmen.

Zu den invarianten Eigenschaften, die durch die Anordnung gebildet werden, zählen insbesondere die invarianten Geraden, die durch die bereits oben erwähnten Geradenbildungspunkte gebildet werden. Hierbei umfassen die Geradenbildungspunkte, die eine der invarianten Geraden bilden, wie erwähnt, vorzugsweise mindestens einen Röntgenmarker, insbesondere mindestens zwei Röntgenmarker.

Invariante Eigenschaften sind hierin insbesondere die bereits erwähnten invarianten Geraden, also Vorrichtungs-Geraden und insbesondere Pyramiden-Geraden. Diesbezüglich gilt auch, dass, falls keine Abbildungsfehler auftreten, die Anzahl der Geraden, die sich in einem Schnittpunkt schneiden, konstant bleibt. Insbesondere ist somit eine Gruppierung der Geradenbildungspunkte, insbesondere der Röntgenmarker nach invarianten Geraden möglich. Eine Gruppierung ist aber auch möglich nach Schnittpunkten, in denen sich die Geraden schneiden, also insbesondere der Vorrichtung-Schnittpunkte und Pyramidenspitzen. Es können somit die Geraden nach Schnittpunkten gruppiert werden, wobei die Geraden wiederum Bildmarker und Röntgenmarker umfassen. Eine weitere invariante Eigenschaft ist die Erhaltung der Nachbarschaftsbeziehungen. Das heißt, sind Röntgenmarker auf einer Vorrichtungs-Geraden, insbesondere Pyramiden-Geraden benachbart, so gilt dies auch für die den Röntgenmarkern entsprechenden Bildmarker auf der der Vorrichtungs-Geraden entsprechenden Bild-Geraden. Dies bedeutet auch, dass eine Abfolge der Geradenbildungspunkte entlang einer Gerade, z.B. ausgehend von einem Schnittpunkt von Geraden erhalten bleibt. Dies bedeutet insbesondere, dass ein Geradenbildungspunkt, der einem Schnittpunkt naheliegt, und ein anderer Geradenbildungspunkt auf derselben Vorrichtungs-Gerade, der weiter weg von dem Schnittpunkt entfernt ist, diese Bedingungen sowohl im dreidimensionalen Raum bezüglich der Anordnung der Röntgenmarker erfüllt, als auch im zweidimensionalen Raum bezüglich der Anordnung der Bildmarker auf der der Vorrichtungs-Geraden entsprechenden Bild-Geraden erfüllt. Befindet sich ein Vorrichtungsmarker 1 und ein Vorrichtungsmarker 2 auf einer Geraden und zwischen den beiden Markern ein Vorrichtungs-Schnittpunkt, aber keine weiteren Röntgenmarker, so zeigt sich im Röntgenbild ein Bildmarker A und ein Bildmarker B, die beidseitig eines Bild-Schnittpunkts liegen. Hierbei ist unklar, ob der Röntgenmarker 1 im Bildmarker A oder B zuzuordnen ist. Unterscheiden sich aber die Anzahl der Röntgenmarker entlang einer Vorrichtungs-Geraden zu beiden Seiden des Vorrichtungs-Schnittpunkts (insbesondere der Pyramidenspitze), so ist eine Zuordnung der Vorrichtungsmarker zu den Bildmarkern möglich. Vorzugsweise unterscheidet sich die Anzahl nicht nur um einen Röntgenmarker, sondern um zwei oder mehr Röntgenmarker, um eine Stabilität gegenüber Artefakten zu gewährleisten. Bei der weiter unten beschriebenen Ausführungsform ist die Anzahl der Röntgenmarker auf einer Seite des Vorrichtungs-Schnittpunktes Null, während sie auf der anderen Seite des Vorrichtungs-Schnittpunktes 2 beträgt.

Eine weitere invariante Eigenschaft ist das o.g. Doppelverhältnis, Insbesondere ist auch eine Gruppierung der Geradenbildungspunkte, insbesondere Röntgenmarker und Bildmarker nach der Zugehörigkeit zu einem Doppelverhältnis möglich.

Allgemein betrachtet, werden somit die Röntgenmarker nach mindestens einer invarianten Eigenschaft gruppiert und die Bildmarker werden nach der entsprechenden mindestens einen invarianten Eigenschaft gruppiert. Diese erfindungsgemäße Gruppierung umfasst insbesondere auch die Gruppierung von Gruppen, die aus einer Gruppierung der Röntgenmarker und/oder Bildmarker nach invarianten Eigenschaften hervorgehen. Beispielsweise werden Röntgenmarker, die auf ein und derselben Vorrichtungs-Geraden liegen, zu einer Vorrichtungs-Geraden-Gruppe zusammengefasst und Bildmarker, die auf einer (ausgewählten) Bild-Geraden liegen, werden zu einer Bild-Geraden-Gruppe zusammengefasst. Ein Beispiel hierfür sind die Geraden-Gruppen, die zu Schnittpunkt-Gruppen gruppiert werden können. Durch diese erfindungsgemäße Gruppierung wird die Anzahl der möglichen Permutationen drastisch verringert. Denn es erfolgt dann erfindungsgemäß eine gruppenweise Zuordnung zwischen den Bildmarkern und den Röntgenmarkern. Diese gruppenweise Zuordnung umfasst insbesondere auch die Zuordnung zwischen Gruppen (z.B. Vorrichtungs-Geraden-Gruppen), die von Röntgenmarkern (oder davon abgeleiteten Gruppen) erfindungsgemäß (also unter Berücksichtigung invarianter Eigenschaften) abgeleitet werden, und Gruppen (z.B. Bild-Geraden-Gruppen), die von Bildmarkern (oder davon abgeleiteten Gruppen) erfindungsgemäß abgeleitet werden. Beispielsweise werden die Röntgenmarker einer Vorrichtungs-Geraden den Bildmarkern einer Bild-Geraden zugeordnet. Damit wird insbesondere die Möglichkeit ausgeschlossen, dass die Röntgenmarker, die auf einer (einzigen) Geraden liegen, Bildmarkern, die auf verschiedenen (mehreren) Geraden liegen, zugeordnet werden können. Wie bereits erwähnt, wird durch die Gruppierung die Anzahl der möglichen Permutationen drastisch verringert. Die erfindungsgemäße Gruppierung erlaubt somit, dass die Berechnung (oder der Versuch der Berechnung) der Abbildungsgeometrie-Daten vorzugsweise nur für Zuordnungsmöglichkeiten erfolgt, die sich nach der erfindungsgemäßen Gruppierung ergeben. Insbesondere ist der erfindungsgemäße Gruppierungsvorgang so gestaltet, dass Gruppen, die aus den elementaren Bestandteilen, d.h. jeweils aus den Röntgenmarkern oder den Bildmarkern hervorgehen, zu ranghöheren Gruppen zusammengefasst werden. Diese ranghöheren Gruppen können dann wiederum zu noch ranghöheren Gruppen zusammengefasst werden. Im gegebenen Beispiel werden die Röntgenmarker zu Vorrichtungs-Geraden-Gruppen zusammengefasst und die Vorrichtungs-Geraden-Gruppen zu Vorrichtungs-Schnittpunkt-Gruppen zusammengefasst. Letztendlich erfolgt dann vorzugsweise die Zuordnung zwischen den Röntgenmarkern und Bildmarkern dergestalt, dass nur diejenigen Röntgenmarker und Bildmarker einander zugeordnet werden, die Bestandteil derselben Gruppen, d.h. der rangniedrigsten Gruppe und aller ranghöheren Gruppen, die die jeweils rangniedrigere Gruppe umfassen, sind. Vorzugsweise werden demnach Zuordnungsmöglichkeiten nach dem Rang der Gruppe sortiert. Es wird davon ausgegangen, dass die Anzahl der Gruppen mit dem höchsten Rang am geringsten ist. Insbesondere ist die Anzahl der höchstrangigen Gruppen, die auf Röntgenmarkern basiert, gleich der Anzahl der höchstrangigen Gruppe, die auf Bildmarkern basiert. Im genannten Beispiel ist also insbesondere die Anzahl der Vorrichtungs-Schnittpunkt-Gruppen gleich der Anzahl der Bild-Schnittpunkt-Gruppen. Ist die Anzahl nicht gleich, wird dies vorzugsweise als ein Hinweis auf Artefakte gewertet. Diese invariante Eigenschaft der gleichen Anzahl der Gruppen gleichen Ranges, die jeweils von den Röntgenmarkern und Bildmarkern abgeleitet sind, gilt insbesondere auch für die niederrangigen Gruppen. Abweichungen von dieser invarianten Eigenschaft der gleichen Anzahl der gleichrangigen Gruppen werden vorzugsweise als Hinweise für Artefakte gewertet. Insbesondere können so auch Artefakte identifiziert werden.

Vorzugsweise werden erfindungsgemäß alle niedrigstrangigen Vorrichtungs-Gruppen und Bild-Gruppen, im genannten Beispiel die Vorrichtungs-Geraden-Gruppen und die Bild-Geraden-Gruppen bestimmt. Gruppenweise werden dann für die niedrigstrangigen Gruppen vorzugsweise die Zuordnungsmöglichkeiten bestimmt. Bei der Bestimmung der Zuordnungsmöglichkeiten werden vorzugsweise die vorgenannte Einteilung der Gruppen in höherrangige und niederrangige Gruppen berücksichtigt. Man greift eine der höchstrangigen Vorrichtungs-Gruppen, im genannten Beispiel eine der Vorrichtungs-Schnittpunkt-Gruppen heraus und ordnet sie einer der höchstrangigen Bild-Gruppen, im genannten Beispiel einer der Bild-Schnittpunkt-Gruppen zu. Die herausgegriffene höchstrangige Vorrichtungs-Gruppe enthält niederrangige Gruppen, im genannten Beispiel Vorrichtungs-Geraden-Gruppen. Auch hiervon greift man wieder eine der niederrangigen Vorrichtungs-Gruppen, im genannten Beispiel also eine der Vorrichtungs-Geraden-Gruppen heraus und ordnet sie einer der niederrangigen Bild-Gruppen, im genannten Beispiel einer der Bild-Geraden-Gruppen zu. Diese Zuordnung wird permutativ für alle Mitglieder der niederrangigen Gruppe (für alle Vorrichtungs-Geraden-Gruppen) der herausgegriffenen höherrangigen Gruppe (Vorrichtungs-Schnittpunkt-Gruppe) zu der jeweiligen niederrangigen Bild-Gruppe (Bild-Geraden-Gruppe) der herausgegriffenen höherrangigen Bild-Gruppe (Bild-Schnittpunkt-Gruppe) permutativ durchgeführt. Hierbei werden insbesondere alle möglichen Pennutationen beachtet, um alle Zuordnungsmöglichkeiten zu bestimmen. Unter gewissen Umständen, kann man jedoch auch hier die Anzahl der möglichen Permutationen verringern. Betrachtet man beispielsweise die Zuordnungsmöglichkeiten zwischen den Mitgliedern einer Vorrichtungs-Geraden-Gruppe und den Mitgliedern einer Bild-Geraden-Gruppe, so kann man hier bei der Bestimmung der Zuordnungsmöglichkeiten auch alle möglichen Permutationen der Zuordnung zwischen den Röntgenmarkern der Vorrichtungs-Geraden-Gruppe und den Bildmarkern der zugeordneten Bild-Geraden-Gruppe bestimmen. Wie im Folgenden erläutert wird, ist es jedoch mit Hilfe der Berücksichtigung der invarianten Eigenschaften möglich, die Anzahl der Zuordnungsmöglichkeiten zu verringern, so dass sie geringer ist, als die Anzahl der möglichen Permutationen. Enthält beispielsweise eine Vorrichtungs-Geraden-Gruppen drei Mitglieder, also drei Röntgenmarker und eine zugeordnete Bild-Geraden-Gruppe ebenfalls drei Mitglieder, also drei Bildmarker, so ergeben sich drei Fakultät mögliche Permutationen, also 6 Zuordnungsmöglichkeiten. Vorzugsweise erfolgt die gruppenweise Bestimmung der Zuordnungsmöglichkeiten unter Berücksichtigung der Zugehörigkeit einer elementaren Gruppe zu einer höher geordneten Gruppe und unter Berücksichtigung der sich daraus ergebenden invarianten Eigenschaften. Zu den berücksichtigten invarianten Eigenschaften gehören insbesondere die bereits oben erwähnten Nachbarschaftsbeziehungen und/oder das Doppelverhältnis. Geht man davon aus, dass eine Vorrichtungs-Geraden-Gruppe beispielsweise vier Röntgenmarker umfasst und diese Vorrichtungs-Gerade-Gruppe einer Bild-Geraden-Gruppe zur Bestimmung der Zuordnungsmöglichkeiten zugeordnet wird, die vorzugsweise die gleiche Anzahl an Bildmarkern, also vier Bildmarker umfasst, so ergeben sich grundsätzlich vier Fakultät, also 24 Möglichkeiten der Zuordnung. Durch die Einordnung der elementaren Gruppe, also der Vorrichtungs-Geraden-Gruppe in höherrangige Gruppen, also der Schnittpunkt-Geraden-Gruppe, ist, wie oben angedeutet, durch die Berücksichtigung invarianter Eigenschaften, eine drastische Reduzierung dieser Zuordnungsmöglichkeiten zwischen elementaren Gruppen möglich. Vorzugsweise werden von den gegebenen möglichen Permutationen nur solche als Zuordnungsmöglichkeiten ausgewählt, die diesen invarianten Eigenschaften genügen. Da die Vorrichtungs-Geraden-Gruppe Mitglied einer Vorrichtungs-Schnittpunkt-Gruppe ist, kann unter Berücksichtigung der invarianten Nachbarschaftsbeziehungen bestimmt werden, welcher der vier Marker dem Schnittpunkt am nächsten liegt, am zweitnächsten liegt, am drittnächsten liegt und am viertnächsten liegt (also am weitesten entfernt ist). Entsprechend kann für die Bild-Geraden-Gruppe vorgegangen werden. Auch dort wird eine Sortierung der Gruppenelemente, also der Bildmarker, nach der Nähe zum Bild-Schnittpunkt vorgenommen. Dies führt dann dazu, dass von den 24 Permutationen nur eine einzige ausgewählt wird. Bei dieser wird der dem Vorrichtungs-Schnittpunkt nächstliegende Röntgenmarker der dem Bild-Schnittpunkt nächstliegenden Bildmarker zugeordnet, der zweitnächstliegende Röntgenmarker dem zweitnächstliegenden Bildmarker zugeordnet, der drittnächstliegende Röntgenmarker dem drittnächstliegenden Bildmarker zugeordnet und der viertnächstliegende Röntgenmarker dem viertnächstliegenden Bildmarker zugeordnet. Bei mehr Röntgenmarkern würde dies entsprechend fortgesetzt werden. Somit kann durch die Verwendung der invarianten Eigenschaft der Nachbarschaftsbeziehung in Verbindung mit der Zugehörigkeit der elementaren Gruppe zu einer höheren Gruppe (die hier den Schnittpunkt festlegt) die Anzahl der durch die Permutationen vorgegebenen Zuordnungsmöglichkeiten drastisch reduziert werden. Wird, wie im gegebenen Beispiel, nur eine Zuordnungsmöglichkeit aus den gegebenen Permutationen ausgewählt, so kann optional weiter das Doppelverhältnis für diese verbliebene Zuordnungsmöglichkeit berechnet werden, wenn mindestens vier Geradenbildungspunkte gegeben sind. Stimmt das Doppelverhältnis für die ausgewählte Zuordnungsmöglichkeit zumindest in einem vorbestimmten Umfang überein, so wird die Zuordnungsmöglichkeit dem nächsten Verfahrensschritt zugeführt. Zum nächsten Verfahrensschritt kann auch direkt ohne Berechnung des Doppelverhältnisses nach Auswahl der Zuordnungsmöglichkeit übergegangen werden. Bei diesem nächsten Verfahrensschritt werden, falls möglich, für die ausgewählte Zuordnungsmöglichkeit Abbildungsgeometrie-Daten berechnet. Stimmt das Doppelverhältnis jedoch nicht überein, so kann gemäß einer Ausführungsform die ausgewählte Zuordnungsmöglichkeit für das weitere Verfahren ausgeschlossen werden. Durch das Ausschließen von Zuordnungsmöglichkeiten lässt sich somit der Rechenaufwand weiter verringern. Denn der Rechenaufwand ist insbesondere für die Berechnung der Abbildungsgeometrie-Daten und der Bestimmung einer Übereinstimmung zwischen einem realen und virtuellen Röntgenbild erheblich.

Für die verbliebenen, insbesondere ausgewählten Zuordnungsmöglichkeiten werden Abbildungsgeometrie-Daten berechnet, die Informationen über die Röntgenstrahlen-Abbildungsgeometrie umfassen, die es insbesondere erlauben, die Lage der Röntgenquelle relativ zu der Röntgenmarkervorrichtung zu berechnen.

Bei dem Versuch der Berechnung der Abbildungsgeometrie-Daten, basierend auf einer der erfindungsgemäß bestimmten Zuordnungsmöglichkeiten wird vorzugsweise ein Algorithmus eingesetzt, der bereits auf den oben erwähnten Prinzipien der Lochkamera beruht. Es wird nochmals auf die oben beschriebenen Zitate diesbezüglich verwiesen.

Können Abbildungsgeometrie-Daten berechnet werden, so werden vorzugsweise virtuelle Röntgenbilder mit Hilfe der Abbildungsgeometrie-Daten und basierend auf der bekannten Anordnung der Röntgenmarker berechnet und die virtuellen Röntgenbilder werden mit dem realen Röntgenbild verglichen.

Ergibt sich bei dem Vergleich eine Übereinstimmung zumindest in einem vorbestimmten Umfang, so wird davon ausgegangen, dass die Abbildungsgeometrie-Daten korrekt sind und somit auch die entsprechende Zuordnungsmöglichkeit korrekt ist. Eine Übereinstimmung in einem vorbestimmten Umfang kann beispielsweise nach einem Least-Square-Fit-Verfahren oder ähnlich bestimmt werden. Beispielsweise können ein virtuelles Röntgenbild und ein reales Röntgenbild (gleicher Größe) überlagert werden und die quadrierten Pixel-Abstände zwischen einem virtuellen Bildmarker und dem zugehörigen realen Bildmarker für jeden Bildmarker bestimmt werden und aufaddiert werden. Ist die Summe unterhalb einer vorbestimmten Grenze, so wird von einer ausreichenden Übereinstimmung ausgegangen, ist sie darüber, dann ist keine ausreichende Übereinstimmung gegeben. Auch kann somit eine Bewertung der verschiedenen Zuordnungsmöglichkeiten erfolgen und diejenige Zuordnungsmöglichkeit gewählt werden, für die die beste Übereinstimmung gegeben ist. Diese Überprüfung kann durch Vergleich weiterer Kenngrößen ergänzt und verfeinert werden.

Bei der Auswertung eines Röntgenbilds werden die Bildmarker identifiziert. Vorzugsweise werden sie aufgrund ihrer Form und/oder Größe und/oder Schwärzungsdichte erkannt. Dennoch kann es vorkommen, dass Bildbereiche, die möglicherweise Bildmarker darstellen und die im Folgenden Bildmarkerkandidaten genannt werden, keine Bildmarker sind. Andererseits kann es vorkommen, dass in Bereichen des Bildes, in denen ein Bildmarker zu sehen sein müsste, dieser nicht oder nicht klar erkennbar ist. Vorzugsweise ist das Verfahren ausgebildet, basierend auf der bekannten Anordnung und insbesondere deren invarianten Eigenschaften, zu bestimmen, ob ein Bildmarkerkandidat ein Bildmarker ist oder nicht. Insbesondere umfasst die Information über die bekannte Anordnung Information über die Anzahl der Röntgenmarker entlang der Vorrichtungs-Geraden. Diese Anzahl stellt ebenfalls eine invariante Eigenschaft dar, da es sich um die Anzahl auf einer Geraden handelt und Geraden eine invariante der projektiven Abbildung darstellen. Die invarianten Eigenschaften ermöglichen es so erfindungsgemäß, Bildmarkerkandidaten als Bildmarker zu erkennen oder zu verwerfen. Insbesondere ist es auch möglich, zu erkennen, wenn an einer bestimmten Stelle ein Bildmarkerkandidat im Bild zu erkennen sein müsste, da dort ein Bildmarker erwartet wird, aber ein derartiger Bildmarkerkandidat nicht vorhanden ist. Allgemein kann man somit, basierend auf den invarianten Eigenschaften Artefakte im Bild erkennen, die insbesondere fehlende Bildmarkerkandidaten sind oder zu verwerfende Bildmarkerkandidaten. Vorzugsweise werden insbesondere Bildmarkerkandidaten als Bildmarker bestimmt, wenn sie sich auf einer Bild-Geraden befinden, auf der sich eine Mindestanzahl von Bildmarkerkandidaten befindet. Diese Mindestanzahl wird durch die Vorrichtungsdaten vorgegeben. Die Mindestanzahl ist gleich der Mindestanzahl der Röntgenmarker auf Vorrichtungsgeraden. Vorzugsweise wird gemäß dem Verfahren davon ausgegangen, dass eine entsprechende Mindestanzahl für Bildmarker auf den Bild-Geraden gilt. Liegen beispielsweise mindestens drei Röntgenmarker auf einer Bild-Geraden, so gilt dies entsprechend für die Bildmarker. Liegen beispielsweise vier Geradenbildungspunkte auf einer Bild-Geraden, die beispielsweise aus einem Schnittpunkt, der einer Pyramidenspitze entspricht (erkennbar durch die invariante Anzahl der sich schneidenden Geraden) und drei Bildmarkerkandidaten besteht, so kann auch mit Hilfe des Doppelverhältnisses überprüft werden, ob es sich bei den vier Bildmarkerkandidaten um Bildmarker handelt. Denn auch das Doppelverhältnis stellt eine invariante Eigenschaft dar, die zur Unterscheidung von korrekten und zu verwerfenden Bildmarkerkandidaten dienen kann.

Hat man im Röntgenbild eine mögliche Bild-Gerade identifiziert, so kann z.B. geprüft werden, ob sich auf dieser möglichen Bild-Geraden dieselbe Anzahl oder Mindestanzahl von Bildmarker befindet, wie sich auf der entsprechenden Vorrichtungs-Geraden Röntgenmarker befinden. Gemäß einer bevorzugten Ausführungsform ist die Anzahl der Röntgenmarker auf den Vornchtungs-Geraden zwei oder mehr als zwei, beispielsweise drei oder vier oder fünf. Die Anzahl der Geradenbildungspunkte ist vorzugsweise drei oder mehr. Erkennt man nun eine Gerade im Röntgenbild, die aus einem Schnittpunkt von Geraden und einem Bildmarkerkandidaten gebildet wird, so kann man gemäß einer bevorzugten Ausführungsform dann diesen Bildmarkerkandidaten als Bildmarker festlegen, wenn sich auf dieser Geraden ein weiterer Bildmarkerkandidat befindet, falls also mindestens eine der aus den Vorrichtungsdaten ableitbaren invarianten Eigenschaften, also beispielsweise die Mindestanzahl der Geradenbildungspunkte auf Vorrichtungs-Geraden und damit auch auf Bild-Geraden erfüllt ist. Vorzugsweise werden also Bildmarkerkandidaten als Bildmarker anerkannt, wenn sie sich auf einer Geraden befinden, auf der sich insgesamt eine Mindestanzahl von Bildmarkern und/oder Bildmarkerkandidaten befindet. Die Mindestanzahl ist insbesondere 2, 3, 4 oder mehr. Die Mindestanzahl entspricht insbesondere der Mindestanzahl der Röntgenmarker auf Vorrichtungs-Geraden.

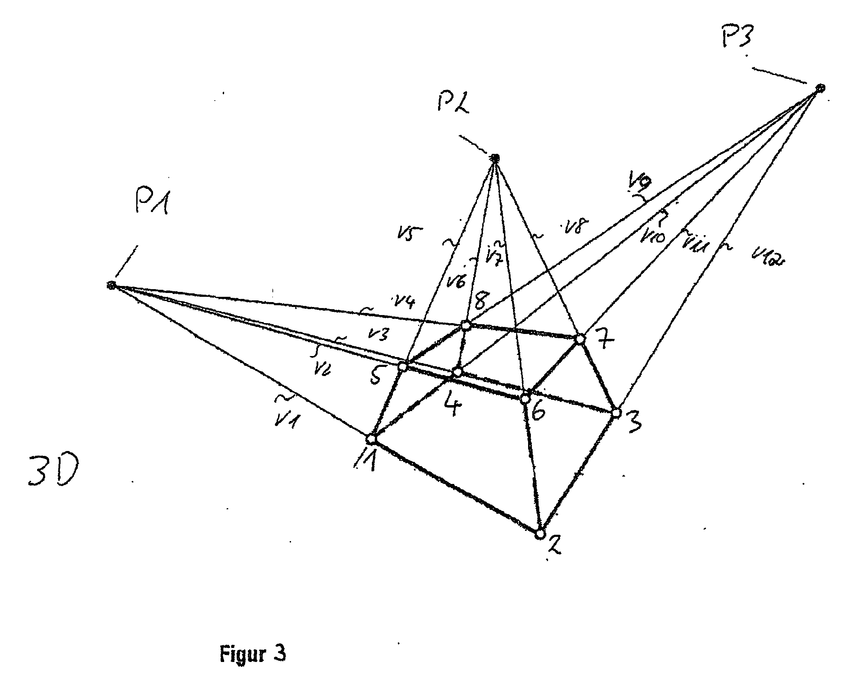

Vorzugsweise werden Bildmarkerkandidaten als Bildmarker bestimmt, wenn sie sich auf einer Bild-Geraden befinden, die sich mit einer vorbestimmten Mindestanzahl von anderen Bild-Geraden schneidet. Auch diese Mindestanzahl ist eine aus den Vorrichtungsdaten bestimmbare invariante Eigenschaft. Bei diesem Beispiel umfasst eine derartige Bild-Gerade mindestens einen Geradenbildungspunkt, der ein Bild-Schnittpunkt ist, in dem sich eine vorbestimmte Mindestanzahl von Bild-Geraden schneidet. Vorzugsweise ist diese Mindestanzahl von Bild-Geraden, die sich in dem Bild-Schnittpunkt schneiden, größer als 2 oder 3, also beispielsweise 3, 4 oder 5. Insbesondere ist der Geradenbildungspunkt nicht identisch mit einem Bildmarker. Insbesondere ist die Anzahl der sich schneidenden Bild-Geraden kleiner als die der minimalen Anzahl der Vorrichtungs-Geraden, die sich am Ort eines beliebigen Röntgenmarkers schneiden. Im gezeigten Beispiel der Figur 3 ist dies die Anzahl der Röntgenmarker minus 1. Auch eine derartige minimale Anzahl ist eine invariante Eigenschaft und kann als Obergrenze für die Identifikation von Bild-Schnittpunkten, die Pyramidenspitzen entsprechen, genutzt werden. Ist also die Anzahl der sich in einem Bild-Schnittpunkt schneidenden Geraden größer gleich der gegebenen Mindestanzahl und kleiner als diese Obergrenze, so kann der Bild-Schnittpunkt als einer Pyramidenspitze entsprechend bestimmt werden.

Vorzugsweise betrifft die Erfindung weiter ein Navigationsverfahren, das eines der vorgenannten erfindungsgemäßen Verfahren zum Bestimmen einer Zuordnung zwischen Röntgenmarkern und Markerbildern umfasst. Gemäß dem erfindungsgemäßen Navigationsverfahren werden nach der erfindungsgemäßen Bestimmung, dass eine der Zuordnungsmöglichkeiten korrekt ist, die der korrekten Zuordnungsmöglichkeit entsprechenden Abbildungsgeometrie-Daten verwendet, um die Lage eines Gegenstandes in dem Röntgenbild zu bestimmen. Bei der Bestimmung der Lage des Gegenstandes in dem (zweidimensionalen) Röntgenbild, wird die dreidimensionale Lage des Gegenstandes zugrunde gelegt. Hierzu werden dem Navigationsverfahren vorzugsweise Gegenstandslagedaten (dreidimensionale Lagedaten) zur Verfügung gestellt, die die Lage des Gegenstands im Raum beschreiben. Diese Lagedaten werden beispielsweise mit Hilfe einer Detektionsvorrichtung detektiert, die Bestandteil eines Navigationssystems ist. Beispielsweise umfasst der Gegenstand eine Navigations-Markervorrichtung, die von der Detektionsvorrichtung detektierbar ist. Ist die dreidimensionale Lage des Gegenstands bekannt und sind die Abbildungsgeometrie-Daten bekannt, so ist ausreichend Information gegeben, um die Lage der Röntgenquelle relativ zu dem Gegenstand berechnen zu können. Diese Information erlaubt somit die Berechnung der Abbildung des Gegenstandes im Röntgenbild. Es kann also virtuell und mit Hilfe von Daten, die das Röntgenbild beschreiben, berechnet werden, wie sich der Gegenstand im Röntgenbild darstellen würde, wenn er im Röntgengerät die Lage eingenommen hätte, die durch die dreidimensionalen Lagedaten (Gegenstandslagedaten) vorgegeben sind. Anders ausgedrückt, wird eine virtuelle Röntgenabbildung des Gegenstandes virtuell unter denselben Bedingungen vorgenommen, wie dies bei der realen Röntgenabbildung der Fall war. Die Lage des Gegenstandes bei dieser virtuellen Röntgenabbildung ist durch die Gegenstandslagedaten vorgegeben. Gemäß einer weiteren Ausführungsform des Navigationsverfahrens, kann die virtuelle Röntgenabbildung auch dann korrekt angezeigt werden, wenn sich das Röntgengerät relativ zu dem Patienten seit Erstellung des realen Röntgenbildes bewegt hat. Bei dieser weiteren Ausführungsform ist eine Navigations-Markervorrichtung am Patienten so angebracht, dass sie vorzugsweise ortsfest relativ zu mindestens einem Teil der in der Röntgenabbildung gezeigten Körperstrukturen ist. Die Abbildungsgeometrie kann somit relativ zu einem Bezugssystem bestimmt werden, in dem die Körperstrukturen ruhen. Insbesondere können die Abbildungsgeometrie-Daten für dieses Bezugssystem bestimmt werden, wie sie zum Zeitpunkt der Erstellung des realen Röntgenbildes gegeben waren. Somit kann auch nach einer Bewegung des Röntgengeräts relativ zum Patienten errechnet werden , wie sich der Gegenstand im Falle einer virtuellen Röntgenabbildung (bei einem virtuell nicht bewegten Röntgengerät) darstellen würde. Insbesondere kann die virtuelle Form und/oder Größe und/oder Lage des Gegenstandes im virtuellen Röntgenbild bestimmt und angezeigt werden.

Auch können gemäß einer weiteren Ausführungsform Navigations-Markervorrichtungen an dem Röntgengerät angebracht werden, insbesondere an der Einheit, die die Röntgenquelle beinhaltet und der Einheit, die den Röntgendetektor beinhaltet. Somit kann durch Auswertung des Röntgenbildes nach dem erfindungsgemäßen Verfahren, also basierend auf den Abbildungsgeometrie-Daten und den Röntgenmarkervorrichtungs-Lagedaten die Abbildungsgeometrie in einem Bezugssystem des Navigationssystems berechnet werden. Insbesondere kann die Lage der Röntgenquelle und die Lage einer Bildebene in dem Bezugssystem und insbesondere relativ zu der an dem Röntgengerät angebrachten Navigations-Markervorrichtung berechnet werden. Auf diese Art und Weise lassen sich nach erfindungsgemäßer Bestimmung der Abbildungsgeometrie-Daten auch ohne die erfindungsgemäße Röntgenmarkervorrichtung, z.B. bei der Untersuchung einer anderen Körperstruktur mittels des Röntgengeräts, virtuelle Röntgenabbildungen errechnen, in denen der Gegenstand virtuell angezeigt wird.

Die vorliegende Erfindung betrifft weiterhin ein Programm, das, wenn es auf einem Computer läuft, den Computer veranlasst die Schritte des erfindungsgemäßen Verfahrens, insbesondere eines der oben beschriebenen Verfahren und Navigationsverfahren durchzuführen. Der Computer umfasst insbesondere einen Prozessor und einen Speicher. Insbesondere sind weiter Schnittstellen vorgesehen, um die die zur Durchführung des Verfahrens notwendigen Informationen als Daten dem Computer zuzuführen. Insbesondere sind dies Daten über die Anordnung der Röntgenmarker in der Röntgenmarkervorrichtung (Vorrichtungsdaten) sowie Daten, die das Röntgenbild (Röntgenbilddaten) beschreiben. Falls das Programm das Navigationsverfahren durchführt, werden weiter vorzugsweise die Gegenstandslagedaten und insbesondere Patientendaten (siehe unten) dem Computer zugeführt. Die Daten, die die Anordnung der Röntgenmarker in der Röntgenmarkervorrichtung beschreiben, werden hierin auch als Vorrichtungsdaten bezeichnet. Die Vorrichtungsdaten umfassen insbesondere Informationen über die relative Lage der Röntgenmarkervorrichtung, wobei die relative Lage durch die Anordnung der Röntgenmarker in der Röntgenmarkervorrichtung vorgegeben ist.

Die Röntgenbilddaten beschreiben das Röntgenbild zumindest teilweise. Insbesondere beschreiben sie die Lage der Markerbilder und/oder von möglichen Markerbildern (Markerbildkandidaten) im Röntgenbild. Die Vorrichtungsdaten umfassen somit Informationen über die relative Lage der Röntgenmarker während die Röntgenbilddaten Informationen über die relative Lage der Bildmarker umfassen. Die relative Lage der Röntgenmarker beschreibt invariante Eigenschaften, die bei einer projektiven Röntgenabbildung erhalten bleiben und die sich in der relativen Lage der Bildmarker widerspiegeln.

Weitere Daten, die dem Computer zugeführt werden können, sind die Patientendaten, die die Lage der im Röntgenbild abgebildeten Körperstruktur beschreiben. Die Patientendaten werden insbesondere mittels des Navigationssystems gewonnen, das eine Navigations-Markervorrichtung detektiert, die ortsfest mit der Körperstruktur verbunden ist.

Das Programm kann auf einem Programmspeicher (z.B. CD oder ROM) gespeichert sein oder in Form einer Signalwelle, die insbesondere über das Internet übertragbar ist, und die Informationen enthält, die das Programm darstellen, dargestellt sein. Die Erfindung ist auch auf diese Speicher oder Darstellungsform, die jeweils das Programm enthalten, gerichtet.

Die Erfindung ist weiter gerichtet auf ein Navigationssystem. Dieses umfasst insbesondere einen Computer, auf den das Programm geladen ist oder läuft. Der Computer umfasst insbesondere einen Prozessor zum Durchführen der Verfahrensschritte und einen Speicher zum Laden des Programms. Als Schnittstelle ist eine Dateneingabeeinrichtung vorgesehen, um die Röntgenbilddaten und die Vorrichtungsdaten in dem Computer einzugeben. Das Navigationssystem umfasst weiter vorzugsweise eine Detektionsvorrichtung, um Navigations-Markervorrichtungen zu detektieren. Vorzugsweise wird eine an der Röntgenmarkervorrichtung angebrachte Navigations-Markervorrichtung detektiert. Vorzugsweise wird auch eine an (mindestens) einem Gegenstand angebrachte Navigations-Markervorrichtung detektiert. Insbesondere wird auch eine, an einer Körperstruktur eines Patienten angebrachte Navigations-Markervorrichtung detektiert. Das Programm, das auf dem Computer des Navigationssystems läuft ist ausgebildet, basierend auf der detektierten Lage der Röntgenmarkervorrichtung und der detektierten Lage des Gegenstandes zu berechnen, wie sich der Gegenstand im Röntgenbild darstellen würde, wenn er gemäß den Abbildungsgeometrie-Daten von Röntgenstrahlen durchstrahlt werden würde, wobei die Abbildungsgeometrie-Daten der als korrekt bestimmten Zuordnung entsprechen. Vorzugsweise sind in einem Speicher Navigations-Markervorrichtungs-Daten abgespeichert, die die relative Lage der verschiedenen detektierten Navigations-Markervorrichtungen zu dem Objekt, an dem die Navigations-Markervorrichtungen angebracht sind, enthalten. Dadurch kann durch Detektion der Lage der Navigations-Markervorrichtung die Lage des Objekts bestimmt werden. Beispielsweise ist also die relative Lage zwischen der Navigations-Markervorrichtung die an dem Gegenstand angebracht ist, und dem Gegenstand abgespeichert. Weiter ist die relative Lage zwischen der Navigations-Markervorrichtung, die an der Röntgenmarkervorrichtung angebracht ist, und der Röntgenmarkervorrichtung abgespeichert. Derartige Daten können, wie beschrieben, abgespeichert sein oder können dem Navigationssystem zugeführt werden, beispielsweise über eine Datenschnittstelle.

### KURZE BESCHREIBUNG DER FIGUREN

- Figur 1: zeigt schematisch den Aufbau eines erfindungsgemäßen Navigationssystems;
- Figur 2: zeigt schematisch die Abbildung einer Röntgenmarkervorrichtung mittels eines Röntgengeräts;
- Figur 3: zeigt schematisch die dreidimensionale Anordnung von Röntgenmarkern einer Röntgenmarkervorrichtung;
- Figur 4: zeigt die relative Lage von Bildmarkern in einem Röntgenbild der Röntgenmarkervorrichtung der Figur 3;
- Figur 5: zeigt die Bestimmung von Bild-Schnittpunkten in dem Röntgenbild die Pyramidenspitzen entsprechen;
- Figur 6: zeigt perspektivisch eine Röntgenmarkervorrichtung;
- Figur 7: zeigt die Vorrichtungs-Schnittpunkte der Röntgenmarkervorrichtung der Figur 6, die Pyramidenspitzen sind.

### AUSFÜHRLICHE BESCHREIBUNG DER FIGUREN

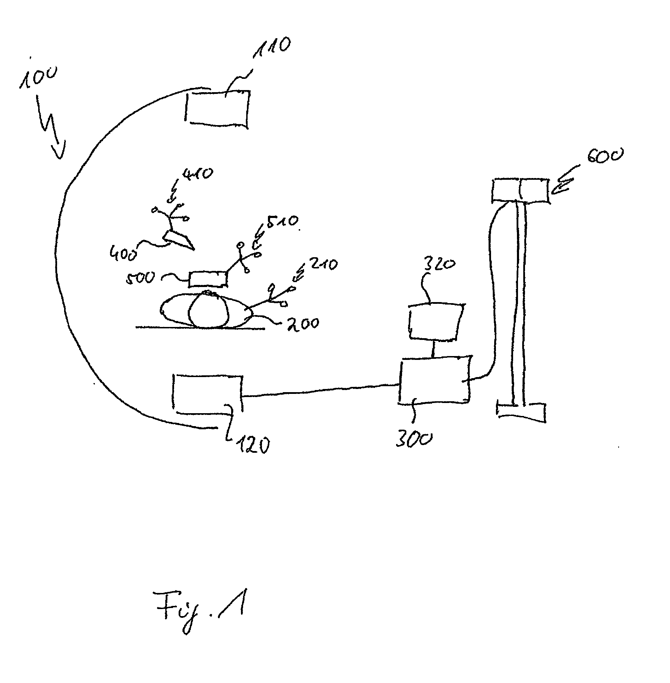

Figur 1 zeigt schematisch ein erfindungsgemäßes Navigationssystem mit einem Röntgengerät 100 (ein C-Bogen), das eine Röntgenquelleneinheit 110 und eine Röntgendetektoreinheit 120 (z.B. Bildverstärker) umfasst. Das Röntgenlicht von der Röntgenquelleneinheit 110 durchstrahlt von oben nach unten einen Gegenstand 400 (z.B. Instrument, insbesondere Skalpell), die erfindungsgemäße Röntgenmarkervorrichtung 500 und den Patienten 200. An dem Gegenstand 400 ist eine Navigations-Markervorrichtung 410 angebracht. An der Röntgenmarkervorrichtung 500 ist eine Navigations-Markervorrichtung 510 angebracht. An dem Patienten 200 ist eine Navigations-Markervorrichtung 210 angebracht. Die Röntgenstrahlen werden in der Röntgendetektoreinheit 120 detektiert und das durch die Röntgendetektoreinheit generierte Röntgenbild 120 wird dem Computer 300 zugeführt. Eine Anzeigeeinrichtung 320, die mit dem Computer 300 verbunden ist, kann Bilddaten, insbesondere zur bildgestützten Navigation oder Röntgenbilder, insbesondere auch das virtuelle Röntgenbild anzeigen. Dem Computer 300 werden weiter Detektionssignale von der Detektionsvorrichtung 600 zugeführt. Die Detektionsvorrichtung 600 detektiert Signale von den Navigations-Markervorrichtungen 410, 510 und 210, insbesondere mittels zweier separater Kameras. Die Detektionssignale der Detektionsvorrichtung 600 werden dem Computer 300 zugeführt, so dass dieser mit einem Navigationsprogramm die Lage des Gegenstandes 400, der Röntgenmarkervorrichtung 500 und des Patienten 200 bestimmen kann. Hierzu wird insbesondere eine in dem Computer enthaltene Datenbank genutzt, in der die relative Lage zwischen den Navigations-Markervorrichtungen und den Objekten, also z.B. Teilen, insbesondere Oberflächen oder Spitzen oder Enden des Gegenstandes 400 und die relative Lage zwischen den Röntgenmarkern und der Navigations-Markervorrichtung 510 abgespeichert sind. Insbesondere unbekannt ist die Lagebeziehung zwischen der Navigations-Markervorrichtung 210 und den Körperstrukturen des Patienten 200, die im Röntgenbild zu sehen sind. Das erfindungsgemäße Navigationssystem erlaubt insbesondere darzustellen, wie der Gegenstand 400 sich im Röntgenbild abbilden würde (insbesondere dessen Spitze), selbst wenn das Röntgengerät 100 vom Patienten entfernt ist und die Röntgenmarkervorrichtung 500 ebenfalls entfernt ist. Bewegt sich der Patient 200 nach der Erzeugung des Röntgenbildes nicht, so wäre die Darstellung des Gegenstandes 400 in einem virtuellen Röntgenbild auch ohne die Navigations-Markervorrichtung 210 möglich. Durch den Einsatz der Navigations-Markervorrichtung 210, die vorzugsweise ortsfest mit den Körperstrukturen verbunden ist, die im Röntgenbild abgebildet werden, ist es möglich, die relative Lage zwischen Gegenstand und Patient auch nach Bewegung des Patienten nach Erzeugung des Röntgenbildes zu bestimmen.

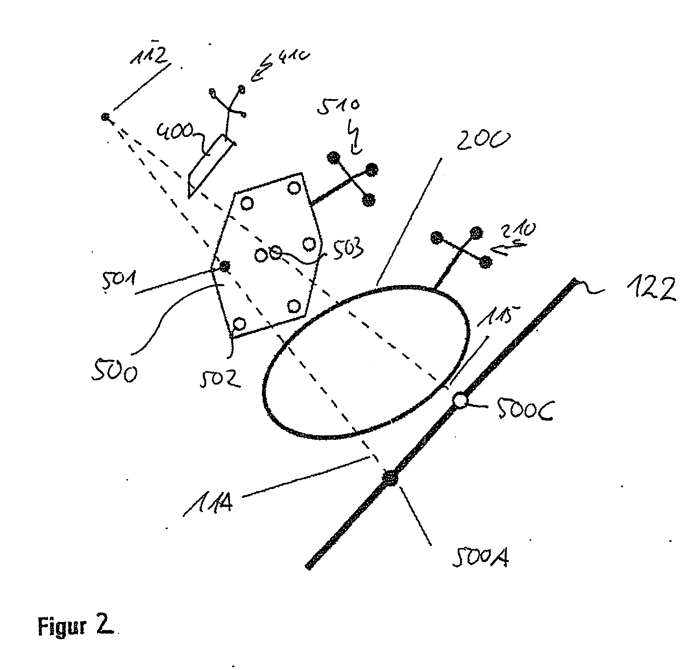

Figur 2 zeigt schematisch eine Röntgenabbildung. Bei dieser Röntgenabbildung wird ein Röntgenbild mittels Röntgenstrahlen erzeugt, die von einer punktförmigen-Röntgenquelle 112 (deren Lage durch das erfindungsgemäße Verfahren errechenbar ist) der Röntgenquelleneinheit 110 ausgehen und auf eine Röntgen-Bildebene 122 der Röntgendetektionseinheit 120 treffen. Auf dem Weg zur Röntgen-Bildebene 122 durchstrahlen die Röntgenstrahlen 114, 115 den Gegenstand 400, die Röntgenmarkervorrichtung 500, die kreisförmig dargestellten Röntgenmarker 501, 502 und weitere nicht mit Bezugszeichen versehene kreisförmige Röntgenmarker umfasst. Danach durchstrahlen die Röntgenstrahlen eine Körperstruktur 200 des Patienten. Mit dieser Körperstruktur ist eine Navigations-Markerstruktur 210 ortsfest verbunden. Der Röntgenmarker 501 erzeugt in einem Röntgenbild, das in der Röntgen-Bildebene 122 liegt, einen Bildmarker 500A. Ein anderer Röntgenmarker 503 erzeugt in dem Röntgenbild einen Bildmarker 500C. Die gestrichelte Linie 114 und 115 bezeichnet schematisch und exemplarisch die Röntgenstrahlen.

Die Erfindung betrifft insbesondere die Zuordnung zwischen Bildmarkern, wie beispielsweise 500A und 500C und Röntgenmarkern, wie beispielsweise 501 und 503. Weiß man, welcher Röntgennarker welchem Bildmarker zuzuordnen ist, so lässt sich hieraus die Lage der punktförmigen Röntgenquelle 112 bestimmen. Ist der Bildmarker 500A dem Röntgenmarker 501 zugeordnet und der Bildmarker 500C dem Röntgenmarker 503, so kann man jeweils eine Gerade durch den Röntgenmarker 501 und dem Bildmarker 500A sowie den Röntgenmarker 503 und dem Bildmarker 500C legen. Die Lage der Röntgenmarker 501 und 503 ist durch Detektion der Navigations-Markervorrichtung 510 bestimmbar. Der Schnittpunkt dieser Geraden stimmt dann mit der Lage der punktförmigen Röntgenquelle überein.

Dies ist nur eine prinzipielle Beschreibung, die mathematischen Details (Algorithmen), die auch die grundsätzlich unbekannte Lage der Röntgen-Bildebene 122, insbesondere Bildmarker 500A und 500C relativ zu der Röntgenmarkervorrichtung 500 berücksichtigen und die Kenntnis dieser Lage zur Berechnung eines virtuellen Röntgenbildes, das sich bei bekannter Lage der Röntgenquelle 112 und der Bildebene 122 ergeben würde, nicht erforderlich machen, sind in den oben beschriebenen Zitaten betreffend des Lochkameraprinzips beschrieben. Die Anwendung dieses mathematischen Details, insbesondere Algorithmen, erlaubt die Bestimmung der Abbildungsgeometrie-Daten, die ausrechend Informationen umfassen, um das virtuelle Röntgenbild zu berechnen. In diesem Zusammenhang wird insbesondere auf die oben erwähnte Veröffentlichungen von Tsai verwiesen. Sie beschreibt eine spezielle Art, die Kalibrierung der Lochkamera zu gewinnen, die oft als Standard angesehen wird. Die Diplomarbeit von Yaniv ist ein Beispiel für die Anwendung einer solchen Kalibrierung auf ein Fluoroskopie-System.

Nimmt man an, dass die Lage der Röntgenquelle bekannt ist, so lässt sich beispielsweise errechnen, welche Bildpunkte der Gegenstand 400 im Röntgenbild erzeugen würde. Insbesondere kann somit berechnet werden, wo und mit welcher Form und/oder Größe der Gegenstand 400 im Röntgenbild abgebildet werden würde. Dies kann insbesondere in Relation zu einer Abbildung der Körperstruktur 200 im Röntgenbild gesetzt werden und in dem virtuellen Röntgenbild, das die Körperstruktur mit dem virtuellen Röntgenbild des Gegenstandes überlagert, gezeigt werden.

Wie oben schematisch anhand der Figur 2 erläutert, ist somit eine korrekte Zuordnung zwischen Röntgenmarkern und Bildmarkern wesentlich. Erfindungsgemäß wird das Auffinden einer korrekten Zuordnung leichter. Wie diese erfindungsgemäß erleichtert wird, wird im Folgenden anhand der Figur 3 erläutert. Figur 3 stellt eine mögliche und bevorzugte Anordnung der Röntgenmarker der Röntgenmarkervorrichtung dar.

Figur 3 zeigt schematisch die dreidimensionale Anordnung von physikalischen Röntgenmarkern 1 bis 8. Die Röntgenmarker 1 bis 8 sind Bestandteil des Registrierkörpers und haben eine ortsfeste Lage zueinander. Die Röntgenmarker liegen an den Seitenkanten dreier Pyramiden, die die Spitzen P1, P2 und P3 haben. Die Spitzen P1, P2 und P3 liegen im dreidimensionalen Raum und sind vorzugsweise virtuell. Die Röntgenmarker 2, 3, 6 und 7 definieren die Grundfläche der Pyramide mit der Spitze P1. Die Röntgenmarker 1, 2, 3 und 4 definieren die Grundfläche der Pyramide mit der Spitze P2 und die Röntgenmarker 1, 2, 5 und 6 definieren die Grundfläche der Pyramide mit der Spitze P3.

Die Lagen der Röntgenmarker sind durch mehrere geometrische Eigenschaften charakterisiert. Insbesondere liegen die Röntgenmarker an Seitenkanten und Grundkanten von Pyramiden. Ist hierin davon die Rede, dass ein Röntgenmarker oder Bildmarker (oder dessen Lage) auf einer Gerade oder Kante oder Spitze oder einem Punkt liegt, so ist hiermit insbesondere gemeint, dass der geometrische Mittelpunkt des Röntgenmarkers oder Bildmarkers diese Bedingung erfüllt. Die Pyramiden haben vorzugsweise mindestens drei Seitenflächen. Im gezeigten Beispiel sind es vier Seitenflächen. Die geometrischen Eigenschaften erleichtern die Zuordnung zwischen den physikalischen Röntgenmarkern 1 bis 8 im dreidimensionalen Raum und den Bildern der Röntgenmarker (im folgenden kurz "Bildmarker") im zweidimensionalen Röntgenbild. Insbesondere können Artefakte erkannt werden und es kann bestimmt werden, dass ein dem physikalischen Röntgenmarker entsprechende Bildmarker fehlt. Bei den in der Figur 3 gezeigten Vorrichtungs-Geraden V1 bis V 12 handelt es sich durchgehend um Pyramiden-Geraden. Die Abschnitte dieser Pyramiden-Geraden, die Pyramiden-Kanten, insbesondere Seitenkanten und/oder Grundkanten bilden, sind in der gezeigten Ausführungsform durch Röntgenmarker begrenzt. Dies ist bevorzugt, aber nicht obligatorisch.

Die vorgenannten geometrischen Eigenschaften sind beispielsweise bei dem Röntgenmarker 1 folgendes: der Röntgenmarker 1 ist Bestandteil der Grundkante (genau an einer Ecke der Grundfläche) der Pyramide mit der Spitze P3. Der Röntgenmarker 1 liegt auf der Pyramiden-Geraden V1, also auf der Pyramiden-Seitenkante mit den Endpunkten Röntgenmarker 2 und Spitze P1 und der Röntgenmarker 1 definiert einen Eckpunkt der Grundfläche der Pyramide mit der Spitze P2. Entsprechende geometrische Eigenschaften, nämlich Ecke der Grundfläche zweier Pyramiden und Bestandteil einer Pyramiden-Seitenkante hat der Röntgenmarker 3.

Der Röntgenmarker 2 liegt an einer Ecke der Grundfläche aller drei Pyramiden (mit Spitzen P1, P2 und P3). Der Röntgenmarker 4 liegt an einer Ecke einer der drei Pyramiden (Pyramide mit Spitze P2) und ist Bestandteil zweier Seitenkanten zweier Pyramiden, nämlich der Seitenkante von 1 nach P3 und von 3 nach P1.

Beispielsweise liegt der Röntgenmarker 8 an keiner einzigen Ecke einer Grundfläche der drei Pyramiden, ist aber Bestandteil dreier Seitenkanten, nämlich der Seitenkante von 7 nach P1 und von 4 nach P2, sowie von 5 nach P3.

Im gezeigten Beispiel ist die Lage eines jeden Röntgenmarkers, vorzugsweise Bestandteil einer Pyramiden-Geraden einer jeden der drei Pyramiden, im gezeigten Beispiel sogar der Kanten der Pyramiden (Seitenkante und/oder Grundkante). Die geometrischen Eigenschaften, genauer Pyramiden-Geraden, die Kanten der Pyramiden enthalten, definieren abbildungsinvariante Eigenschaften, nämlich Geraden, die bei den perspektivischen Abbildungen (Röntgenabbildung) invariant bleiben, d.h. wieder auf Geraden abgebildet werden.

Insgesamt haben die Lagen aller Röntgenmarker also mehrfache geometrische und insbesondere invariante Eigenschaften. Im gezeigten Beispiel liegen sie jeweils auf drei invarianten Geraden, die bei der späteren Berechnung im Algorithmus eine Zuordnung zwischen den (physikalischen, dreidimensionalen) Röntgenmarkern und den (zweidimensionalen) Bildmarkern ermöglichen. Eine weitere invariante Eigenschaft sind die Nachbarschaftsbeziehungen der Röntgenmarker, so ist der Marker 1 der Pyramidenspitze P1 näher als der Marker 2 und der Marker 5 der Pyramidenspitze P2 näher als der Marker 1. Derartige Eigenschaften, die die Röntgenmarker nach ihrer Nähe zu den Pyramidenspitzen sortieren, bleiben ebenfalls erhalten, d.h. im Bild ergibt sich dieselbe Sortierung nach der Nähe zu den Bild-Schnittpunkten, die den Pyramidenspitzen entsprechen.

Insbesondere durch die Nutzung der invarianten Eigenschaften wird es ermöglicht, den Algorithmus effizient zu gestalten, um die Rechenzeit drastisch zu verkürzen.

Wie bereits oben ausgeführt unterliegen die Röntgenmarker bei der Erzeugung des Röntgenbildes geometrisch betrachtet einer Projektion, infolge dessen das zweidimensionale Bild mit den Bildmarkern erzeugt wird. Die Röntgenbilder können verzerrt sein, falls früher übliche Bildverstärker mit gekrümmter Detektionsfläche verwendet wurden, oder verzerrungsfrei sein, falls neuere Flach-Panel-Bildverstärker genutzt werden. Hierin wird davon ausgegangen, dass verzerrte Röntgenbilder nach üblichem Verfahren entzerrt werden. Die aus den Röntgenbildern abgeleiteten und weiterverarbeiteten Röntgenbilddaten sind also vorzugsweise verzerrungsfrei. Wie ebenfalls erwähnt wurde, ist ein wesentlicher Aspekt der Erfindung, dass geometrische Eigenschaften der Markeranordnung ausgenutzt werden, die bei einer geometrischen Projektion invariant sind. Zu den bereits oben aufgezählten Eigenschaften zählt dabei insbesondere, dass Geraden auf Geraden abgebildet werden, die hierin auch als invariante Geraden bezeichnet werden. Wie oben ausgeführt, ist vorzugsweise jeder der Röntgenmarker an einer Pyramidenecke oder an einer Seitenkante zwischen einer Grundflächenecke und der Pyramidenspitze angeordnet und somit Bestandteil einer Pyramiden-Gerade die durch drei Punkte definiert ist oder der Röntgenmarker ist an einer Ecke einer Pyramide angeordnet und somit ebenfalls Bestandteil einer Pyramiden-Gerade, die durch die Pyramidenspitze und einem weiteren Röntgenmarker zwischen der Pyramidenspitze und der Grundflächenecke definiert ist. Insgesamt gehen somit durch einen Röntgenmarker im gezeigten Beispiel drei Pyramiden-Geraden, die durch jeweils einen anderen Röntgenmarker und eine der Pyramidenspitze definiert sind. Da, wie erwähnt, durch eine Projektion Geraden auf Geraden abgebildet werden, wird das Auffinden von Bildmarkern im zweidimensionalen Bild, denen physikalische Röntgenmarker zugeordnet werden können, erleichtert und beschleunigt.

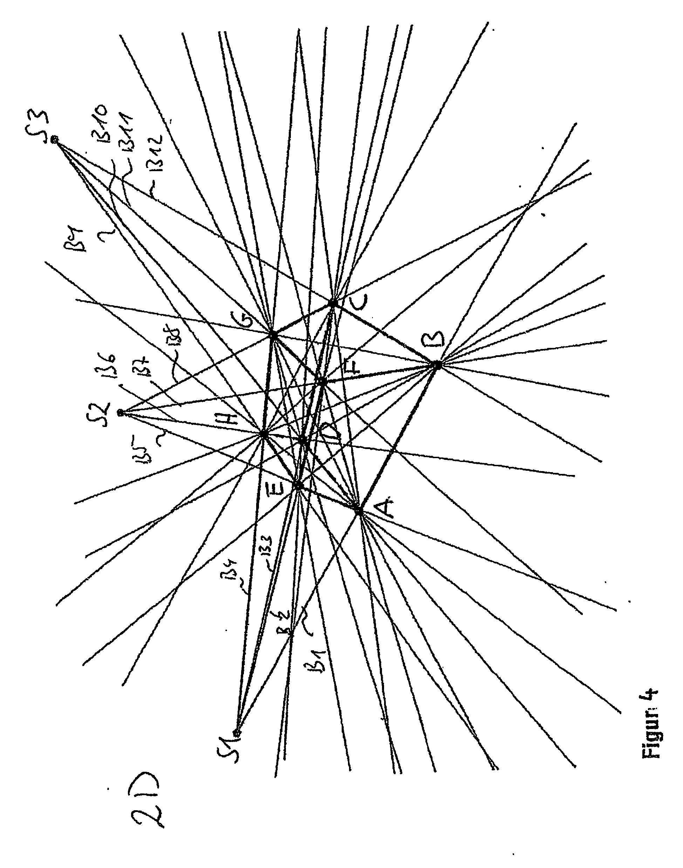

Die Röntgenmarker 1 bis 8 können sich in einem Röntgenbild durch Bildmarker A, B, C, D, E, F, G und H zeigen. Diese sind durch schwarze Kreisflächen in Figur 4 gezeigt. Gemäß dem erfindungsgemäßen Verfahren soll nun geklärt werden, welcher der Röntgenmarker 1 bis 8 welchem der Bildmarker A bis H zuzuordnen ist. Insbesondere werden hierzu in dem zweidimensionalen Röntgenbild gemäß Figur 4 vorzugsweise alle Bildmarker A bis H miteinander durch Bild-Geraden verbunden. In jedem Bildmarker schneiden sich somit sieben Geraden (Anzahl der Bildmarker minus 1). Neben diesen Bild-Schnittpunkten gibt es noch weitere Bild-Schnittpunkte, wobei sich üblicherweise nur zwei Geraden in einem Punkt schneiden (siehe Figur 4). Abgesehen von diesen Bild-Schnittpunkten gibt es jedoch noch die Schnittpunkte S1, S2 und S3, in denen sich mehr als zwei Geraden, genauer vier Geraden schneiden. Diese Bild-Geraden B1 bis B12 entsprechen den Pyramiden-Geraden V1 bis V12.

Da sich bei einer geometrischen Projektion Geraden auf Geraden abbilden, werden somit auch Schnittpunkte von Geraden auf Schnittpunkte von Geraden abgebildet. Da sich in den Pyramidenspitzen P1, P2 und P3 ebenfalls jeweils vier Geraden schneiden, kann man davon ausgehen, dass es sich bei den Bild-Schnittpunkten S1, S2 und S3 um Bilder der Pyramidenspitzen P1, P2 und P3 handelt. Hierbei ist jedoch noch unklar, welche Pyramidenspitze P1, P2, P3 welchem Schnittpunkt S1, S2, S3 zuzuordnen ist. Die Anzahl der sich in den Bild-Schnittpunkten schneidenden Geraden liegt also zwischen der minimalen Anzahl, der sich in einem Bildmarker schneidenden Geraden (Anzahl der Röntgenmarker minus 1) und der sich maximal zufällig in einem Schnittpunkt schneidenden Geraden (Anzahl typischerweise gleich zwei, vielleicht und sehr selten drei, je nach Genauigkeit der Bildauflösung). Die Röntgenmarkervorrichtung wird demnach vorzugsweise so gestaltet, dass die Anzahl der sich in einer Pyramidenspitze schneidenden Pyramiden-Geraden diese Bedingungen erfüllen.

Die Schnittpunkte S1, S2 und S3, die Pyramidenspitzen entsprechen, erlauben ein geordnetes Suchen nach einer Zuordnung zwischen dem Bildmarker A bis H und den physikalischen Röntgenmarkern 1 bis 8. Die Anzahl der möglichen Zuordnung kann erheblich durch die Kenntnis reduziert werden, dass die Schnittpunkte S1, S2 und S3 Bilder der Pyramidenspitzen P1, P2 und P3 darstellen. Ohne ein derartiges geordnetes Suchen nach der korrekten Zuordnung, basierend auf den geometrischen Eigenschaften der Anordnung der physikalischen Röntgenmarker, insbesondere unter Berücksichtigung der projektionsinvarianten geometrischen Eigenschaften der Anordnung wäre das Auffinden der korrekten Zuordnung erheblich zeitaufwändiger. Für 8 Markerkugeln gibt es acht Fakultät verschiedene Möglichkeiten der Zuordnungen, für die ein Kandidaten-Projektionsbild berechnet werden müsste und dann dieses mit dem tatsächlichen Röntgenbild verglichen werden müsste. Im Falle von 8 Markerkugeln müssten also 8 Fakultät und somit 40.320 Kandidaten-Projektionsbilder berechnet und mit dem tatsächlichen Bild verglichen werden. Bei Anwesenheit von Artefakten im Bild, die noch nicht von den Markerbildern unterschieden werden können, steigt der kombinatorische Aufwand weiter an, da auch Kombinationen betrachtet werden müssen, die eines oder mehrere Artefakte enthalten. Dieser Rechenaufwand kann erheblich durch das folgende an einem Beispiel beschriebene Zuordnungsverfahren verkürzt werden.

Hierbei nutzt man die Invarianz bei perspektivischen Abbildungen aus, dass Geraden auf Geraden abgebildet werden. Liegt beispielsweise eine Pyramidenspitze am Schnittpunkt von n Geraden, hat als n Seitenkanten, so ergibt sich im Röntgenbild ein Schnittpunkt von genau n Geraden, wenn alle Bildmarker gefunden wurden und keine Artefakte zu betrachten sind. Werden nicht alle Bildmarker gefunden, die die Geraden bilden, die sich im Schnittpunkt schneiden, der der Pyramidenspitze entspricht, so können sich im Schnittpunkt weniger n Geraden schneiden. Durch die Abbildung von Geraden auf Geraden bleiben auch die Nachbarschaftsbeziehungen zwischen Punkten auf Geraden erhalten. So liegt beispielsweise in Figur 3 der Röntgenmarker 5 zwischen dem Punkt P1 und dem Röntgenmarker 6. Damit liegt im Röntgenbild das Bild vom Röntgenmarker 5 (also Bildmarker 5) ebenfalls zwischen dem durch Schnittpunktbildung gefundenen Bild von Punkt P1 und dem Bild vom Röntgenmarker 6 (also Bildmarker 6) auf der durch das Bild vom Punkt P1 und Bild vom Röntgenmarker 6 definierten Gerade. Bei der beschriebenen Ausführungsform existieren die Pyramidenspitzen nur konstruktiv aber nicht physikalisch. Auch liegt der Röntgenmarker 4 zwischen dem Röntgenmarker 1 und dem Punkt P3. Somit liegt das Bild vom Röntgenmarker 4 zwischen dem Bild vom Röntgenmarker 1 und dem Bild vom Punkt P3 auf der durch das Bild vom Röntgenmarker 1 und das Bild vom Punkt P3 definierten Gerade. Nutzt man dies aus, so kann man wie folgt vorgehen:

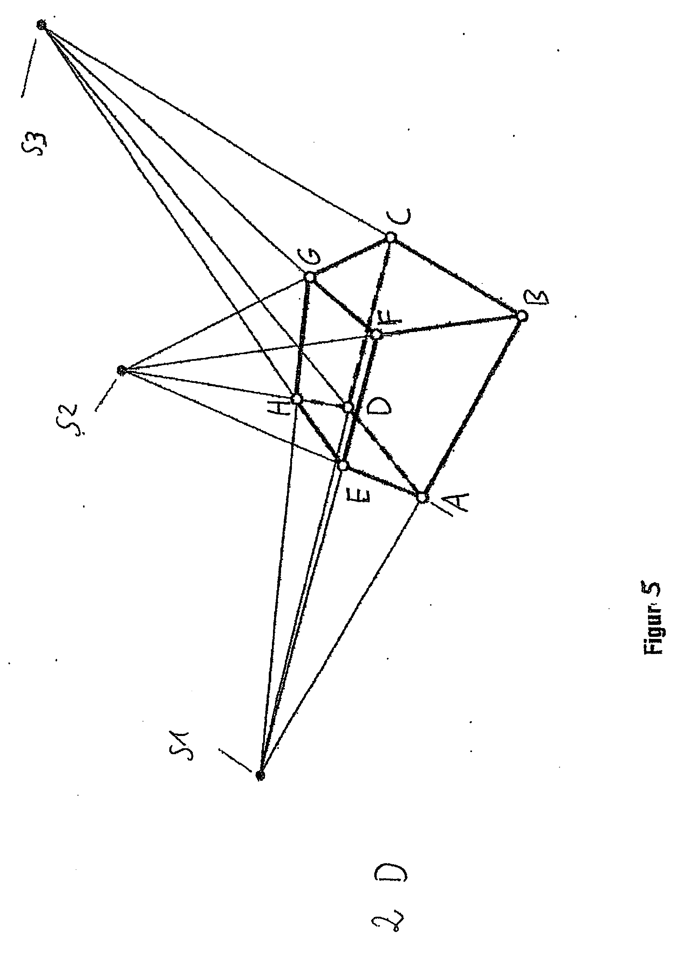

Nutzt man die invarianten Eigenschaften aus, so kann man diejenigen Bild-Geraden ausschließen, die keine Pyramiden-Geraden entsprechen, so dass sich aus der in Figur 4 gezeigten Situation die übersichtlichere, in Figur 5 gezeigte Situation ergibt, anhand der das weitere Verfahren erläutert wird.

Man nimmt als erstes an, dass der Schnittpunkt S1 im zweidimensionalen Bild mit der Pyramidenspitze P1 übereinstimmt.

S1 ast der Schnittpunkt von Geraden, die durch die folgenden Bildmarker-Paare definiert sind: (A, B); (E, F); (H, G); (D, C). Um die vorgenannte erhaltene Nachbarschaftsbeziehung zu nutzen, wurde oben in den runden Klammern zuerst dasjenige Bildmarker genannt, das dem Schnittpunkt S1 am nächsten ist und dann das weiter entfernt liegende.

Vergleicht man die Situation in Figur 4 mit der Figur 3 und folgt man der oben getroffenen Annahme, dass der Schnittpunkt S1 der Pyramidenspitze P1 entspricht, so wären die durch die physikalischen Marker definierten Geraden wie folgt: (1, 2); (5, 6); (8, 7); (4, 3).

Auch bei der eben aufgeführten Bezeichnung der Geraden wurde jeweils derjenige physikalische Röntgenmarker zuerst genannt, der dem Punkt P1 am nächsten ist.

In dem oben genannten Beispiel gibt es maximal 4!, also 24 Möglichkeiten, um den vier Bildmarker-Paaren (A, B); (E, F); (H, G); (D, C) jeweils ein Bildmarker-Paar aus den physikalischen Röntgenmarker-Paaren (1, 2); (5, 6); (8, 7); (4, 3) zuzuordnen. Man kann hier auch von 24 möglichem bijektiven Abbildungen zwischen der Menge der Bildmarker-Paare und der Menge der Röntgenmarker-Paare sprechen. Wird beispielsweise dem Bildmarker-Paar (A, B) das physikalische Markerpaar (5, 6) zugeordnet, so bedeutet dies, dass das Bildmarker A dem physikalischen Röntgenmarker 5 zugeordnet wird und das Bildmarker, B dem physikalischen Röntgenmarker 6. Eine Zuordnung des Markerbildes A zu dem physikalischen Röntgenmarker 6 und das Bildmarker B zu dem physikalischen Röntgenmarker 5 ist nicht möglich, da, wie oben ausgeführt wurde, die Nachbarschaftsbeziehungen erhalten bleiben. Somit wird jeweils dem ersten Bildmarker eines Bildmarker-Paares der erste physikalische Röntgenmarker eines physikalischen Markerpaares zugeordnet und das zweite Bildmarker eines Bildmarker-Paares dem zweiten physikalischen Röntgenmarker eines physikalischen Markerpaares zugeordnet. Dies grenzt die Anzahl der Möglichkeiten stark ein.

Die vorgenannten Permutationen der bijektiven Abbildungen zwischen den physikalischen Röntgenmarker-Paaren und den Bildmarker-Paaren stellen jeweils Lösungsmöglichkeiten dar. Für jede Lösungsmöglichkeit (bijektive Abbildung) wird mittels eines Algorithmus, der auf dem bereits oben erwähnten, bekannten Prinzip der Lochkamera beruht, nach einer mathematischen Lösung des Problems gesucht. Das heißt, es wird geprüft, ob für die gegebene Lösungsmöglichkeit Abbildungsgeometrie-Daten, die Informationen über Abbildungsparameter des Kameramodells, d.h. die externen Abbildungsparameter, die insbesondere die Lage und Orientierung der Strahlungsquelle im Weltkoordinatensystem (insbesondere relativ zu den Röntgenmarkern) beschreiben, sowie die internen Abbildungsparameter enthalten, mit Algorithmen berechnet werden können. Dabei kann sich ergeben, dass keine Daten mit dem Algorithmus berechenbar sind. Dies bedeutet dann, dass die Lösungsmöglichkeit falsch ist. Es können sich aber auch Daten berechnen lassen. Ob diese Daten eine korrekte Lösung darstellen, kann dann überprüft werden. Die Daten enthalten Information über die Lage der Strahlungsquelle relativ zu der dreidimensionalen Anordnung der Röntgenmarker sowie über weitere interne Abbildungsparameter, entsprechend dem gewählten Kameramodell. Sie erlauben somit eine virtuelle Durchstrahlung der dreidimensionalen Markeranordnung, um so virtuell ein zweidimensionales Bild mit Bildmarkern zu erzeugen. Stimmt die relative Lage der virtuellen Bildmarker in diesem virtuellen zweidimensionalen Bild zumindest innerhalb eines vorgegebenen Fehlerrahmens mit der relativen Lage der realen Bildmarker in dem realen zweidimensionalen Röntgenbild überein, so ist eine akzeptable Lösungsmöglichkeit gefunden. Ist dies nicht der Fall, so ist die Lösungsmöglichkeit zu verwerfen.

Das oben genannte Verfahren, um zu überprüfen, ob eine Lösungsmöglichkeit (Permutation) korrekt ist, wird, zumindest falls keine richtige (korrekte) Lösungsmöglichkeit gefunden wurde, für alle Lösungsmöglichkeiten durchgeführt oder abgebrochen, falls eine richtige (korrekte) Möglichkeit gefunden wurde. Vorzugsweise werden alle weiteren möglichen Kombinationen untersucht, um gegebenenfalls eine noch bessere Lösungsmöglichkeit zu finden. Die beste Lösungsmöglichkeit wird dann als die korrekte Zuordnung angesehen. Es ist möglich, die Bewertung einer gefundenen Lösungsmöglichkeit zu erweitern, indem noch andere typische Eigenschaften der errechneten Abbildung betrachtet werden, z.B. bestimmte Werte oder Verhältnisse der internen Abbildungsparameter.

Konnte für die möglichen Permutationen betreffend einen Schnittpunkt (im gegebenen Beispiel der Schnittpunkt S1) keine richtige Lösungsmöglichkeit gefunden werden, so schreitet das erfindungsgemäße Verfahren zur nächsten Annahme fort. Gemäß der nächsten Annahme entspricht der Schnittpunkt S1 der Pyramidenspitze P2.

Für diese Konstellation gibt es folgende mögliche physikalische Markerpaare: (5, 1), (6, 2), (7, 3), (8, 4).

Diesen physikalischen Markerpaaren können dann in der bereits oben beschriebenen Weise als Lösungsmöglichkeiten die oben genannten Bildmarker-Paare zugeordnet werden. Es gibt somit wiederum 4!, also 24 Möglichkeiten.

Falls dies wiederum zu keiner Lösung führt, wird im nächsten Schritt dann angenommen, dass der Schnittpunkt S1 der Pyramidenspitze P3 entspricht und analog wiederum für 4!, also 24 möglichen Permutationen nach der Lösung gesucht.

Vorzugsweise beginnt man mit demjenigen Schnittpunkt, der am weitesten von den Bildmarker in der Bildebene entfernt liegt. Bei dem in Figur 4 gezeigten Beispiel wäre dies S3. Führt eine Zuordnung von S3 zu P1, P2 und P3 nicht zum Erfolg, so würde man dann zum nächstgelegenen Schnittpunkt S1 übergehen. Für diesen Fall wurden die obigen Permutationen erläutert. Es wurde also nacheinander angenommen, dass der Schnittpunkt S1 dem Pyramidenpunkt P1, P2 und letztendlich P3 entspricht, wie dies oben beispielhaft beschrieben wurde. Als letztes würde dann mit Hilfe von S2 nach einer Lösungsmöglichkeit gesucht werden, indem angenommen wird, dass der Schnittpunkt S2 nacheinander dem Pyramidenpunkt P1, P2 oder P3 entspricht.

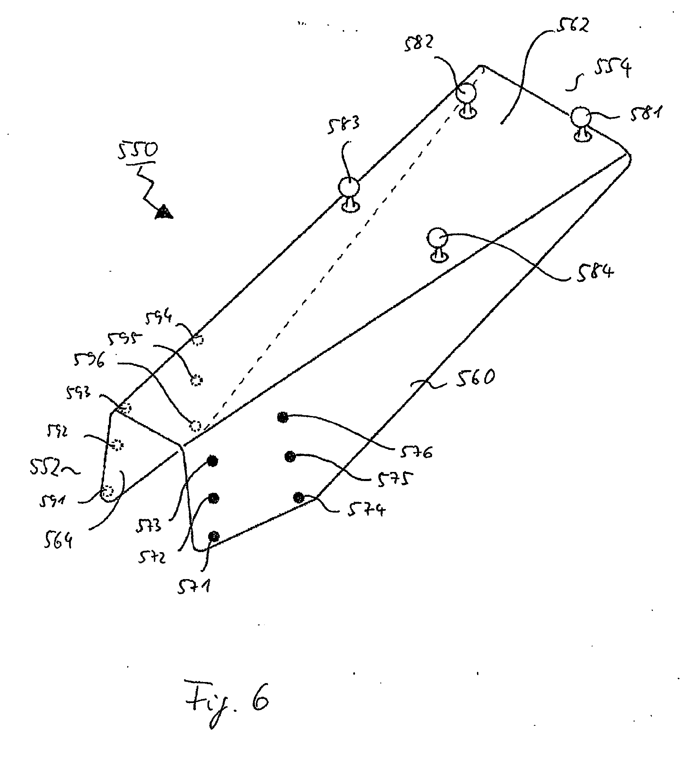

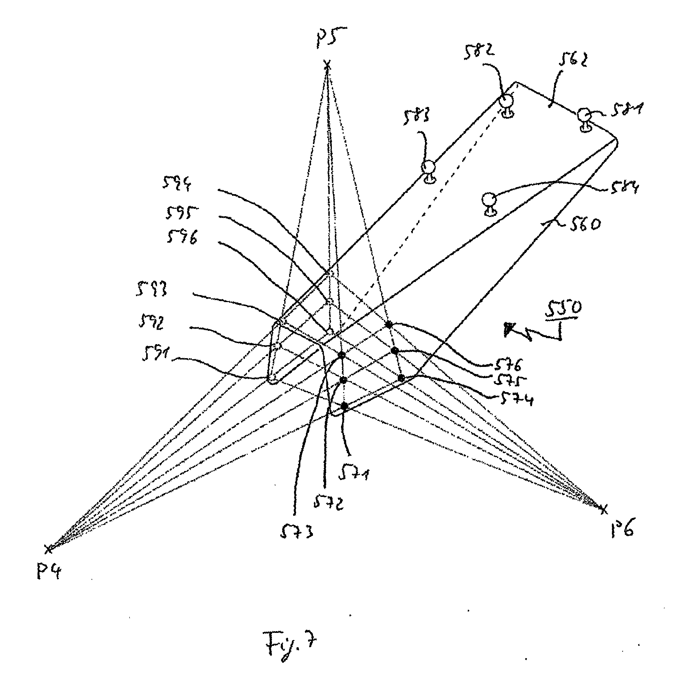

Figur 6 zeigt eine Ausführungsform 550 einer Röntgenmarkervorrichtung. Diese Ausführungsform ist länglich gestaltet mit einem sich von einem in Figur 6 gezeigten linken Ende 552 bis zu einem rechten Ende 554 verbreiternden U-förmigen Querschnitt. In Richtung der Verbreiterung ist rechts die Seitenwand 560, die einen Arm des U's darstellt. In der Mitte ist eine mittlere, in der Figur 6 obenliegende Seitenwand 562 und links ist eine Seitenwand 564. Die linke und rechte Seitenwand entfernen sich immer weiter voneinander, je weiter sie von der mittleren Seitenwand entfernt sind. Die Röntgenmarkervorrichtung umfasst vorzugsweise ein für Röntgenstrahlen vergleichsweise durchlässiges Material, z.B. Karbon, das als Träger für die Röntgenmarker 571, 572, 573, 574, 575 und 576 sowie für die Navigationsmarker 581, 582, 583 und 584 dient. Insbesondere sind die Seitenwände aus diesem Material gebildet. In den insbesondere dünnen Seitenwänden (Wanddicke kleiner als Durchmesser der Röntgenmarker) sind vorzugsweise die Röntgenmarker eingelassen. Die Seitenwände und/oder die Röntgenmarker sind insbesondere optisch opak. Die Navigationsmarker 581 - 584 bilden zusammen eine Navigations-Markervorrichtung. Die Navigationsmarker sind in der gezeigten Ausführungsform an der mittleren Seitenwand 562 angeordnet. In Längsrichtung der Röntgenmarkervorrichtung befinden sie sich an einem anderen Ende als die Röntgenmarker. Vorzugsweise werden sie bei diesen oder bei anderen Ausführungsformen, insbesondere außerhalb eines Bereiches angeordnet, der durch die Röntgenmarker begrenzt ist. Zu dem bereits erwähnten Röntgenmarkern 571 und 576, die von der rechten Seitenwand 560 getragen werden, gibt es weitere entsprechende 6 Röntgenmarker 591-596, die in Fig. 6 als gepunktete Kreise angedeutet sind. Diese entsprechenden 6 Röntgenmarker sind, wenn man die mittlere Seitenwand 562 durch eine senkrechte Ebene (nicht gezeigt) teilt, spiegelbildlich zu dieser Ebene in der Seitenwand 564 angeordnet. Die Anordnung der insgesamt 12 Röntgenmarker bildet eine Pyramidenstumpfanordnung, ähnlich des in Figur 3 gezeigten Anordnung. Die jeweils außen liegenden Marker von drei in einer Reihe angeordneten Markern, liegen jeweils auf der Seitenkante von drei Pyramiden. Wie die Pyramiden gebildet werden, ist in Figur 7 zu sehen. Die Spitzen der drei Pyramiden sind mit P4, P5 und P6 bezeichnet. Beispielsweise treffen sich Pyramidengeraden, die durch die Röntgenmarkerpaare (573, 576), (572,575), (571, 574); (591, 596), (592, 595), und (593, 594) gebildet werden, in der Pyramidenspitze P4.

Vorzugsweise ist insbesondere in dem Bereich, in dem sich die Röntgenmarker befinden, die Seitenwand (linke, mittlere oder rechte Seitenwand) zumindest teilweise unterbrochen, um so eine Dämpfung der Röntgenstrahlen möglichst gering zu halten.

Die oben beschriebene Erfindung bezog sich beispielhaft auf den Fall der Erzeugung eines Bildes (Röntgenbild) von einer Markervorrichtung (Röntgenmarkervorrichtung) mittels einer Abbildungsvorrichtung (Röntgengerät), die eine Abbildung (Röntgenabbildung) gemäß den Gesetzen der linearen Abbildung erzeugt. Allgemein betrifft die Beschreibung den Fall der Erzeugung eines Strahlenbildes von einer Strahlenmarkeranordnung mittels einer Strahlenabbildungsvorrichtung, die eine Strahlenabbildung gemäß den Gesetzen der linearen Abbildung erzeugt. Die Strahlenabbildungsvorrichtung umfasst eine Strahlenquelle (z.B. Röntgenquelle oder Lichtquelle) und einen Strahlendetektor (z.B. Röntgendetekor oder Lichtdetektor), der das Strahlenbild der Strahlenmarkervorrichtung detektiert. Die vorliegende Erfindung bezieht sich somit allgemein auf den Einsatz beliebiger elektromagnetischer Strahlen, wie insbesondere Infrarotstrahlen, sichtbarer Lichtstrahlen und ultravioletter Strahlen. Allgemein betrifft somit die vorliegende Erfindung eine Strahlenmarkervorrichtung mit Strahlenmarkern (z.B. Röntgenmarkervorrichtung oder Lichtmarkervorrichtung), die elektromagnetische Strahlen eines bestimmen Typs (z.B. Röntgenstrahlen oder Lichtstrahlen) nicht durchlassen oder zumindest stark dämpfen. Betrifft die Strahlenmarkervorrichtung sichtbares Licht, so können beispielsweise Lichtmarker, d.h. opake Marker (z.B. kugelförmig) von einem Träger aus für diesen Typ von elektromagnetischer Strahlung transparenten Material (z.B. Glas) getragen werden und erfindungsgemäß angeordnet sein. Allgemein gefasst betrifft die vorliegende Beschreibung somit folgende Vorrichtung:

Strahlenmarkervorrichtung mit einer Anordnung von Strahlenmarkern, wobei die Anordnung Geraden definiert, die als Vorrichtungs-Geraden bezeichnet werden, wobei zumindest ein Teil der Vorrichtungs-Geraden, die als Pyramiden-Geraden bezeichnet werden, Abschnitte aufweisen, die Kanten zumindest einer Pyramide definieren.

Entsprechend betrifft die Beschreibung allgemein folgendes Verfahren:
Verfahren zum Bestimmen einer Zuordnung zwischen Strahlenmarkern einer Strahlenmarkervorrichtung und Markerbildern eines Strahlenbildes, das sich bei einer gegebenen Abbildungsgeometrie durch eine Strahlen-Abbildung der Strahlenmarkervorrichtung ergibt, wobei die Markerbilder die Strahlenmarker in dem Strahlenbild darstellen,
und wobei neben Strahlenbilddaten, die Informationen über die relative Lage der Markerbilder im Strahlenbild umfassen, Vorrichtungsdaten bereitgestellt werden, die die relative Lage der Strahlenmarker beschreiben, und wobei die relative Lage der Strahlenmarker eine Anordnung beschreibt, die invariante Eigenschaften aufweist, die bei der Strahlen-Abbildung erhalten bleiben und die invariante Geraden umfassen,
wobei das Verfahren folgende Schritte umfasst:
   a) basierend auf den Strahlenbilddaten und den Vorrichtungsdaten werden Markerbilder und Strahlenmarker jeweils unter Berücksichtigung der invarianten Eigenschaften der Anordnung gruppiert;
   b) gruppenweise werden die Zuordnungsmöglichkeiten zwischen Markerbildern und Strahlenmarkern bestimmt;
   c) falls für die Zuordnungsmöglichkeiten Abbildungsgeometrie-Daten für die Strahlenabbildung berechenbar sind, werden basierend auf den berechneten Abbildungsgeometrie-Daten virtuelle Strahlenbilder bestimmt und die virtuellen Strahlenbilder werden mit dem realen Strahlenbild verglichen;
   d) wird eine Übereinstimmung zwischen einem bestimmten virtuellen und einem realen Strahlenbild zumindest in einem vorbestimmten Umfang festgestellt, so wird die dem bestimmten Strahlenbild zugrunde liegende Zuordnungsmöglichkeit als korrekt erkannt.

Allgemein betrifft die Beschreibung natürlich auch ein Programm, das, wenn es auf einem Computer läuft, den Computer veranlasst, die Schritte des oben genannten Verfahrens durchzuführen.

Allgemein kann die hierin beschriebene Offenbarung so aufgefasst werden, dass der Begriff "Röntgen" durch "Strahlen" ersetzt wird. Dabei bezeichnet der Begriff "Strahlen" irgendeine Form der elektromagnetischen Strahlung. Das Strahlenbild ist das Bild, das durch diese Strahlung erzeugt wird. Ein Beispiel für das Strahlenbild ist das Röntgenbild oder ein durch Licht erzeugtes Bild.

## Patentansprüche

1. Röntgenmarkervorrichtung (550) mit einer Anordnung von Röntgenmarkern (571, ..., 576; 591, ..., 596), wobei die Anordnung Geraden definiert, die als Vorrichtungs-Geraden bezeichnet werden,
**dadurch gekennzeichnet, dass**
zumindest ein Teil der Vorrichtungs-Geraden, die als Pyramiden-Geraden bezeichnet werden, Abschnitte aufweisen, die die Kanten von Pyramiden definieren, wobei mindestens eine Spitze jeder der Pyramiden außerhalb eines durch die Röntgenmarker (571, ..., 576; 591, ..., 596) begrenzten Bereichs liegt.

2. Röntgenmarkervorrichtung (550) nach Anspruch 1, bei welcher auf mindestens drei der Pyramiden-Geraden, die sich in der Spitze der mindestens einen Pyramide schneiden, insgesamt mindestens 5 der Röntgenmarker (571, ..., 576; 591, ..., 596) liegen.

3. Röntgenmarkervorrichtung (550) nach einem der vorhergehenden Ansprüche, wobei entlang mindestens drei der Pyramiden-Geraden jeweils mindestens zwei der Röntgenmarker (571, ..., 576; 591, ..., 596) angeordnet sind.

4. Röntgenmarkervorrichtung (550) nach einem der vorhergehenden Ansprüche, die eine Navigations-Markervorrichtung (581, ..., 584) zur Detektion der Lage der Röntgenmarkervorrichtung (550) durch ein Navigationssystem umfasst, wobei die Navigations-Markervorrichtung (581, ..., 584) außerhalb eines durch die Röntgenmarker (571, ..., 576; 591, ..., 596) begrenzten Bereichs liegt.

5. Verfahren zum Bestimmen einer Zuordnung zwischen Röntgenmarkern (571, ..., 576; 591, ..., 596) einer Röntgenmarkervorrichtung (550) nach einem der vorhergehenden Ansprüche und Markerbildern eines Röntgenbildes, das sich bei einer gegebenen Abbildungsgeometrie durch eine Röntgen-Abbildung der Röntgenmarkervorrichtung (550) ergibt, wobei die Markerbilder die Röntgenmarker (571, ..., 576; 591, ..., 596) in dem Röntgenbild darstellen,
und wobei neben Röntgenbilddaten, die Informationen über die relative Lage der Markerbilder im Röntgenbild umfassen, Vorrichtungsdaten bereitgestellt werden, die die relative Lage der Röntgenmarker (571, ..., 576; 591, ..., 596) beschreiben, und wobei die relative Lage der Röntgenmarker (571, ..., 576; 591, ..., 596) eine Anordnung beschreibt, die invariante Eigenschaften aufweist, die bei der Röntgen-Abbildung erhalten bleiben und die invariante Geraden umfassen, wobei die Anordnung Geraden definiert, die die invariante Eigenschaft haben und als Vorrichtungs-Geraden (V1, ..., V 12) bezeichnet werden,
wobei das Verfahren folgende Schritte umfasst:
a) basierend auf den Röntgenbilddaten und den Vorrichtungsdaten werden Markerbilder und Röntgenmarker (571, ..., 576; 591, ..., 596) jeweils unter Berücksichtigung der invarianten Eigenschaften der Anordnung gruppiert, wobei Markerbilder, die auf einer gemeinsamen, als Bild-Gerade bezeichneten Geraden liegen, jeweils zu einer Bild-Geraden-Gruppe zusammengefasst werden und Röntgenmarker (571, ..., 576; 591, ..., 596), die auf einer der Vorrichtungs-Geraden (V1, ..., V12) liegen, jeweils zu einer Vorrichtungs-Geraden-Gruppe zusammengefasst werden;
b) gruppenweise werden die Zuordnungsmöglichkeiten zwischen Markerbildern und Röntgenmarkern bestimmt, wobei zur Bestimmung der Zuordnungsmöglichkeiten zwischen den Röntgenmarkern (571, ..., 576; 591, ..., 596) und Bildmarkern (A, ..., H) bestimmt wird, welche Zuordnungen zwischen den Bild-Geraden-Gruppen und den Vorrichtungs-Geraden-Gruppen möglich sind;
c) falls für die Zuordnungsmöglichkeiten Abbildungsgeometrie-Daten für die Röntgen-Abbildung, die die Geometrie der Röntgen-Abbildung beschreiben, unter Berücksichtigung der invarianten Eigenschaften berechenbar sind, werden basierend auf den berechneten Abbildungsgeometrie-Daten virtuelle Röntgenbilder der Röntgenmarker bestimmt und die virtuellen Röntgenbilder werden mit einem realen Röntgenbild der Röntgenmarker verglichen;
d) wird eine Übereinstimmung zwischen einem bestimmten virtuellen und einem realen Röntgenbild zumindest in einem vorbestimmten Umfang festgestellt, so wird die dem bestimmten virtuellen Röntgenbild zu Grunde liegende Zuordnungsmöglichkeit als korrekt erkannt.

6. Verfahren nach Anspruch 5, bei welchem der Schritt der Gruppierung umfasst, dass
Vorrichtungs-Geraden-Gruppen, die auf Vorrichtungs-Geraden basieren, die sich in einem gemeinsamen Vorrichtungs-Schnittpunkt schneiden, zu Vorrichtungs-Schnittpunkt-Gruppen zusammengefasst werden und
Bild-Geraden-Gruppen, die auf Bild-Geraden basieren, die sich in einem gemeinsamen Bild-Schnittpunkt schneiden, zu Bild-Schnittpunkt-Gruppen zusammengefasst werden,

7. Verfahren nach Anspruch 6, bei welchem der Schritt der gruppenweisen Bestimmung der Zuordnungsmöglichkeiten umfasst, dass
jeweils für eine der Zuordnungsmöglichkeiten zwischen den Vorrichtungs-Schnittpunkten und den Bild-Schnittpunkten, die Zuordnung von den Röntgenmarkern (571, ..., 576; 591, ..., 596), die Mitglieder einer der Vorrichtungs-Geraden-Gruppen sind, zu den Bildmarkern (A, ..., H), die Mitglieder einer der Bild-Geraden-Gruppe sind, so erfolgt, dass die Röntgenmarker (571, ..., 576; 591, ..., 596) und die Bildmarker (A, ..., H) einander zugeordnet werden, die jeweilig in einer Richtung entlang der Vorrichtungs-Gerade (V1, ..., V12) ausgehend vom Vorrichtungs-Schnittpunkt, im Falle der Röntgenmarker (571, ..., 576; 591, ..., 596), und in einer Richtung entlang der Bild-Gerade ausgehend vom Bild-Schnittpunkt, im Falle der Bildmarker (A, ..., H) denselben Rang in der Abfolge einnehmen, wobei die Anzahl der Bildmarker (A, ..., H) der Abfolge und die Anzahl der Röntgenmarker (571, ..., 576; 591, ..., 596) der Abfolge gleich sind und wobei die Anzahl der Röntgenmarker (571, ..., 576; 591, ..., 596) auf der Vorrichtungs-Geraden (V1, ..., V12), wenn man ausgehend vom Vorrichtungs-Schnittpunkt in die entgegengesetzte Richtung geht, anders ist.

8. Verfahren nach einem der Ansprüche 5 bis 7, bei welchem bei dem Schritt der gruppenweisen Bestimmung der Zuordnungsmöglichkeiten invariante Eigenschaften so berücksichtigt werden, dass durch Ausschluss von Zuordnungsmöglichkeiten die Anzahl der durch Permutation möglichen Zuordnungen reduziert werden.

9. Verfahren nach einem der Ansprüche 5 bis 8, bei welchem basierend auf mindestens einer der invarianten Eigenschaften der Anordnung, die aus den Vorrichtungsdaten bestimmt wird, Bildbereiche im Röntgenbild, die möglicherweise Bildmarker (A, ..., H) darstellen, als Bildmarker (A, ..., H) bestimmt werden, und/oder bestimmt wird, dass in einem Bildbereich die Darstellung eines Bildmarkers (A, ..., H) fehlt.

10. Navigationsverfahren, umfassend das Verfahren nach einem der Ansprüche 5 bis 9, bei welchem Gegenstandlagedaten, die die Lage eines Gegenstandes (400) beschreiben, und Röntgenmarkervorrichtung-Lagedaten, die die Lage der Röntgenmarkervorrichtung (550) beschreiben, bereitgestellt werden; und
bei welchem basierend auf den Abbildungsgeometrie-Daten, die der als korrekt erkannten Zuordnungsmöglichkeit entsprechen, den Röntgenmarkervorrichtungs-Lagedaten und den Gegenstandslagedaten, die den Abbildungsgeometrie-Daten entsprechende, projektive Abbildung des Gegenstands (400) im Röntgenbild berechnet wird.

11. Programm, das, wenn es auf einem Computer (300) läuft, den Computer (300) veranlasst, die Schritte nach einem der Verfahren 5 bis 10 durchzuführen.

12. Navigationssystem mit einem Computer (300), auf dem das Programm nach Anspruch 11 geladen ist oder läuft,
wobei der Computer (300) eine Dateneingabeeinrichtung aufweist, um die Röntgenbilddaten und die Vorrichtungsdaten einzugeben,
mit einer Detektionsvorrichtung (600), um Navigations-Markervorrichtungen (210, 410, 510) zu detektieren, um die Lage der Röntgenmarkervorrichtung (571, ..., 576; 591, ..., 596) und eines Gegenstandes (400) zu bestimmen;
mit einer Anzeigevorrichtung (320),
wobei das Programm ausgebildet ist, basierend auf der detektierten Lage der Röntgenmarkervorrichtung (571, ..., 576; 591, ..., 596) und des Gegenstandes (400) zu berechnen, wie der Gegenstand (400) im Röntgenbild dargestellt werden würde, wenn es gemäß denjenigen Abbildungsgeometrie-Daten von Röntgenstrahlen durchstrahlt werden würde, die der als korrekt bestimmten Zuordnung entspricht.

## Claims

1. An x-ray marker device (550) comprising an arrangement of x-ray markers (571, ..., 576; 591, ..., 596), wherein the arrangement defines straight lines which are referred to as device straight lines,
**characterised in that**
at least some of the device straight lines, which are referred to as pyramid straight lines, comprise portions which define the edges of pyramids, wherein at least one tip of each of the pyramids lies outside a region which is delineated by the x-ray markers (571, ..., 576; 591, ..., 596).

2. The x-ray marker device (550) according to claim 1, wherein a total of at least five of the x-ray markers (571, ..., 576; 591, ..., 596) lie on at least three of the pyramid straight lines which intersect in the tip of the at least one pyramid.

3. The x-ray marker device (550) according to any one of the preceding claims, wherein at least two of the x-ray markers (571, ..., 576; 591, ..., 596) are respectively arranged along at least three of the pyramid straight lines.

4. The x-ray marker device (550) according to any one of the preceding claims, which comprises a navigation marker device (581, ..., 584) for detecting the position of the x-ray marker device (550) using a navigation system, wherein the navigation marker device (581, ..., 584) lies outside a region which is delineated by the x-ray markers (571, ..., 576; 591, ..., 596).

5. A method for determining an assignment between x-ray markers (571, ..., 576; 591, ..., 596) of an x-ray marker device (550) according to any one of the preceding claims and marker images of an x-ray image which results from x-ray imaging of the x-ray marker device (550) with a given imaging geometry, wherein the marker images represent the x-ray markers (571, ..., 576; 591, ..., 596) in the x-ray image,
and wherein in addition to x-ray image data, which comprise information concerning the relative position of the marker images in the x-ray image, device data are provided which describe the relative position of the x-ray markers (571, ..., 576; 591, ..., 596), and wherein the relative position of the x-ray markers (571, ..., 576; 591, ..., 596) describes an arrangement which exhibits invariant characteristics which are retained in x-ray imaging and comprise invariant straight lines, wherein the arrangement defines straight lines which have the invariant characteristic and are referred to as device straight lines (V1, ..., V12),
wherein the method comprises the following steps:
a) on the basis of the x-ray image data and the device data, marker images and x-ray markers (571, ..., 576; 591, ..., 596) are respectively grouped by taking into account the invariant characteristics of the arrangement, wherein marker images which lie on a common straight line referred to as an image straight line are respectively combined to form an image straight line group, and
x-ray markers (571, ..., 576; 591, ..., 596) which lie on one of the device straight lines (V1, ..., V12) are respectively combined to form a device straight line group;
b) the possible assigmnents between marker images and x-ray markers are determined in groups, wherein in order to determine the possible assignments between the x-ray markers (571, ..., 576; 591, ..., 596) and image markers (A, ..., H), a determination is made as to which assignments between the image straight line groups and the device straight line groups are possible;
c) if imaging geometry data for x-ray imaging which describe the x-ray imaging geometry can be calculated for the possible assignments by taking into account the invariant characteristics, virtual x-ray images of the x-ray markers are determined on the basis of the calculated imaging geometry data, and the virtual x-ray images are compared with an actual x-ray image of the x-ray markers;
d) if a match between a determined virtual x-ray image and an actual x-ray image to at least a predetermined extent is determined, then the possible assignment on which the determined virtual x-ray image is based is recognised as being correct.

6. The method according to claim 5, wherein the grouping step comprises the step that device straight line groups which are based on device straight lines which intersect in a common device intersection point are combined to form device intersection point groups, and
image straight line groups which are based on image straight lines which intersect in a common image intersection point are combined to form image intersection point groups.

7. The method according to claim 6, wherein the step of determining the possible assignments in groups comprises the step that
for each one of the possible assignments between the device intersection points and the image intersection points, x-ray markers (571, ..., 576; 591, ..., 596) which are members of one of the device straight line groups are assigned to the image markers (A, ..., H) which are members of one of the image straight line groups, such that those x-ray markers (571, ..., 576; 591, ..., 596) and image markers (A, ..., H) are assigned to each other which respectively assume the same rank in the sequence in a direction along the device straight line (V1, ..., V12) starting from the device intersection point in the case of the x-ray markers (571, ..., 576; 591, ..., 596), and in a direction along the image straight line starting from the image intersection point in the case of the image markers (A, ..., H), wherein the number of image markers (A, ..., H) of the sequence and the number of x-ray markers (571, ..., 576; 591, ..., 596) of the sequence are equal, and wherein the number of x-ray markers (571, ..., 576; 591, ..., 596) on the device straight line (V1, ..., V12), starting from the device intersection point and going in the opposite direction, is different.

8. The method according to any one of claims 5 to 7, wherein in the step of determining the possible assignments in groups, invariant characteristics are taken into account such that the number of assignments which are possible due to permutation is thus reduced by excluding possible assigmnents.

9. The method according to any one of claims 5 to 8, wherein on the basis of at least one of the invariant characteristics of the arrangement determined from the device data, image regions in the x-ray image which may represent image markers (A, ..., H) are determined as image markers (A, ..., H), and/or it is determined that the representation of an image marker (A, ..., H) is missing in an image region.

10. A navigation method, comprising the method according to any one of claims 5 to 9, wherein object position data which describe the position of an object (400), and x-ray marker device position data which describe the position of the x-ray marker device (550), are provided, and
wherein on the basis of the imaging geometry data which correspond to the possible assignment which has been recognised as being correct, the x-ray marker device position data and the object position data, the projective image of the object (400) in the x-ray image which corresponds to the imaging geometry data is calculated.

11. A program which, when it is running on a computer (300), causes the computer (300) to perform the steps according to any one of methods 5 to 10.

12. A navigation system comprising: a computer (300) on which the program according to claim 11 is loaded or is running,
wherein the computer (300) comprises a data input device for inputting the x-ray image data and the device data;
a detection device (600) for detecting navigation marker devices (210, 410, 510) in order to determine the position of the x-ray marker device (571, ..., 576; 591, ..., 596) and an object (400);
and a display device (320),
wherein the program is designed to calculate, on the basis of the detected position of the x-ray marker device (571, ..., 576; 591, ..., 596) and the object (400), how the object (400) would be displayed in the x-ray image if it were irradiated with x-ray beams in accordance with the imaging geometry data which correspond to the assignment which has been determined to be correct.

## Revendications

1. Dispositif marqueur de rayons X (550) avec un agencement de marqueurs de rayons X (571, ..., 576 ; 591, ..., 596), dans lequel l'agencement définit des droites qui sont désignées comme des droites de dispositif,
**caractérisé en ce qu'**au moins une partie des droites de dispositif, qui sont appelées des droites de pyramide, incluent des portions qui définissent les bords de pyramides, au moins un sommet de chacune des pyramides se situant à l'extérieur d'une zone délimitée par les marqueurs de rayons X (571, ..., 576 ; 591, ..., 596).

2. Dispositif marqueur de rayons X (550) selon la revendication 1, dans lequel un total d'au moins 5 des marqueurs de rayons X (571, ..., 576 ; 591, ..., 596) est situé sur au moins trois des droites de pyramide qui se coupent dans le sommet de la au moins une pyramide.

3. Dispositif marqueur de rayons X (550) selon l'une des revendications précédentes, dans lequel au moins deux des marqueurs de rayons X (571, ..., 576 ; 591, ..., 596) sont respectivement agencés le long d'au moins trois des droites de pyramide.

4. Dispositif marqueur de rayons X (550) selon l'une des revendications précédentes, lequel dispositif inclut un dispositif marqueur de navigation (581, ..., 584) pour détecter la position du dispositif marqueur de rayons X (550) par un système de navigation, le dispositif marqueur de navigation (581, ..., 584) étant à l'extérieur d'une zone délimitée par les marqueurs de rayons X (571, ..., 576 ; 591, ..., 596).

5. Procédé pour déterminer une affectation entre des marqueurs de rayons X (571, ..., 576 ; 591, ..., 596) d'un dispositif marqueur de rayons X (550) selon l'une des revendications précédentes et des images de marqueurs d'une image de rayons X qui résulte d'une imagerie de rayons X du dispositif marqueur de rayons X (550) avec une géométrie d'imagerie donnée, les images de marqueurs représentant les marqueurs de rayons X (571, ..., 576 ; 591, ..., 596) dans l'image de rayons X,
et dans lequel en plus de données d'image de rayons X qui incluent des informations concernant la position relative des images de marqueurs dans l'image de rayons X, sont fournies des données de dispositif qui décrivent la position relative des marqueurs de rayons X (571, ..., 576 ; 591, ..., 596), et dans lequel la position relative des marqueurs de rayons X (571, ..., 576 ; 591, ..., 596) décrit un agencement présentant des caractéristiques invariantes qui restent maintenues lors de l'imagerie de rayons X et qui incluent des droites invariantes, l'agencement définissant des droites qui ont la caractéristique invariante et qui sont désignées droites de dispositif (V1, ..., V12),
le procédé comportant les étapes suivantes consistant à :
a) sur la base des données d'image de rayons X et des données de dispositif, regrouper respectivement des images de marqueurs et des marqueurs de rayons X (571, ..., 576 ; 591, ..., 596) en tenant compte des caractéristiques invariantes de l'agencement, les images de marqueurs qui sont sur l'une des droites communes appelées droite d'image, étant respectivement regroupées dans un groupe de droites d'image et les marqueurs de rayons X (571, ..., 576 ; 591, ..., 596) qui sont sur l'une des droites de dispositif (V1, ..., V12) sont respectivement regroupés dans un groupe de droites de dispositif,
b) déterminer de manière groupée les possibilités d'affectation entre des images de marqueurs et des marqueurs de rayons X, les possibilités d'affectation entre les marqueurs de rayons X (571, ..., 576 ; 591, ..., 596) et les marqueurs d'image (A, ..., H), étant déterminées en déterminant les affectations possibles entre les groupes de droites d'image et les groupes de droites de dispositif,
c) si des données de géométrie d'imagerie pour l'imagerie de rayons X, qui décrivent la géométrie de l'imagerie de rayons X, peuvent être calculées en tenant compte des caractéristiques invariantes pour les possibilités d'affectation, déterminer des images de rayons X virtuelles des marqueurs de rayons X sur la base des données de géométrie d'imagerie calculées, et comparer les images de rayons X virtuelles avec une image de rayons X réelle des marqueurs de rayons X,
d) si une correspondance entre une image de rayons X virtuelle déterminée et une image de rayons X réelle est déterminée au moins sur une étendue prédéterminée, reconnaître alors que la possibilité d'affectation basée sur l'image de rayons X virtuelle déterminée est correcte.

6. Procédé selon la revendication 5, dans lequel l'étape du groupement inclut les étapes consistant à :
combiner en groupes de points d'intersection de dispositif des groupes de droites de dispositif qui sont basés sur des droites de dispositif qui se coupent en un point d'intersection de dispositif commun,
combiner en groupes de points d'intersection d'image des groupes de droites d'image qui sont basés sur des droites d'image qui se coupent en un point d'intersection d'image commun.

7. Procédé selon la revendication 6, dans lequel l'étape de détermination des possibilités d'affectation de manière groupée inclut l'étape consistant à :
pour chacune des possibilités d'affectation entre les points d'intersection de dispositif et les points d'intersection d'image, affecter les marqueurs de rayons X (571, ..., 576 ; 591, ..., 596) qui sont des éléments de l'un des groupes de droites de dispositif, aux marqueurs d'image (A, ..., H) qui sont des éléments de l'un des groupes de droites d'image, de telle sorte que les marqueurs de rayons X (571, ..., 576 ; 591, ..., 596) et les marqueurs d'image (A, ..., H) sont affectés les uns aux autres, lesquels occupent respectivement le même rang dans la séquence dans une direction le long de la droite de dispositif (V1, ..., V12) en partant du point d'intersection de dispositif, dans le cas des marqueurs de rayons X (571, ..., 576 ; 591, ..., 596), et dans une direction le long de la droite d'image en partant du point d'intersection d'image, dans le cas des marqueurs d'image (A, ..., H), dans lequel le nombre des marqueurs d'image (A, ..., H) de la séquence et le nombre des marqueurs de rayons X (571, ..., 576 ; 591, ..., 596) de la séquence sont égaux, et dans lequel le nombre des marqueurs de rayons X (571, ..., 576 ; 591, ..., 596) sur les droites de dispositif (V1, ..., V12), en partant du point d'intersection de dispositif et en allant dans la direction opposée, est différent.

8. Procédé selon l'une des revendications 5 à 7, dans lequel à l'étape de la détermination des possibilités d'affectation de manière groupée, des caractéristiques invariantes sont prises en compte de telle sorte qu'en excluant les possibilités d'affectation, le nombre des affectations possibles par permutation est réduit.

9. Procédé selon l'une des revendications 5 à 8, dans lequel sur la base d'au moins une des caractéristiques invariantes de l'agencement qui est déterminé à partir des données de dispositif, des zones d'image dans l'image de rayons X, qui représentent peut-être des marqueurs d'image (A, ..., H), sont déterminées comme étant des marqueurs d'image (A, ..., H), et/ou on détermine que la représentation d'un marqueur d'image (A, ..., H) est manquante dans une zone d'image.

10. Procédé de navigation comportant le procédé selon l'une des revendications 5 à 9, dans lequel des données de position d'objet, qui décrivent la position d'un objet (400), et des données de position de dispositif de marqueur de rayons X, qui décrivent la position du dispositif de marqueur de rayons X (550), sont fournies, et
dans lequel sur la base des données de géométrie d'imagerie, qui correspondent à la possibilité d'affectation déterminée comme étant correcte, des données de position de dispositif de marqueur de rayons X et des données de position d'objet, l'imagerie de projection de l'objet (400) dans l'image de rayons X, qui correspond aux données de géométrie d'imagerie, est calculée.

11. Programme qui, lorsqu'il s'exécute sur un ordinateur (300), amène l'ordinateur (300) à exécuter les étapes de l'un des procédés selon les revendications 5 à 10.

12. Système de navigation comportant :
un ordinateur (300), sur lequel le programme selon la revendication 11 est chargé ou exécuté, l'ordinateur (300) comportant un dispositif d'entrée de données afin d'entrer les données d'image de rayons X et les données de dispositif,
un dispositif de détection (600) afin de détecter des dispositifs marqueurs de navigation (210, 410, 510) pour déterminer la position du dispositif marqueur de rayons X (571, ..., 576 ; 591, ..., 596) et d'un objet (400),
un dispositif d'affichage (320),
dans lequel le programme est conçu pour calculer, sur la base de la position détectée du dispositif marqueur de rayons X (571, ..., 576 ; 591, ..., 596) et de l'objet (400), la manière dont l'objet (400) serait représenté dans l'image de rayons X s'il était traversé par des faisceaux de rayons X conformément aux données de géométrie d'imagerie, qui correspondent à l'affectation déterminée comme étant correcte.
